# EUROPÄISCHE PATENTSCHRIFT

(11) **EP 2 197 886 B1**
(45) Veröffentlichungstag und Bekanntmachung des Hinweises auf die Patenterteilung: **26.12.2012**
(21) Anmeldenummer: 08802398.1
(22) Anmeldetag: 19.09.2008
(51) Int. Cl.: C07D 495/04, A61K 31/4365, A61P 35/00, A61P 35/02

(54) **5-CYANO-THIENOPYRIDINE ZUR BEHANDLUNG VON TUMOREN**
5-CYANO-THIENOPYRIDINES FOR THE TREATMENT OF TUMORS
5-CYANO-THIÉNOPYRIDINES UTILISÉES DANS LE TRAITEMENT DE TUMEURS

(30) Priorität: 16.10.2007 DE 102007049451
(43) Veröffentlichungstag der Anmeldung: 23.06.2010
(73) Patentinhaber: Merck Patent GmbH, 64293 Darmstadt (DE)
(72) Erfinder: HOELZEMANN, Guenter, 64342 Seeheim-Jugenheim (DE); GRAEDLER, Ulrich, 69469 Weinheim (DE); GREINER, Hartmut, 64331 Weiterstadt (DE); AMENDT, Christiane, 64367 Muehltal/Trautheim (DE); MUSIL, Djordje, 64342 Seeheim-Jugenheim (DE); HILLERTZ, Per, 64289 Darmstadt (DE)
(86) Internationale Anmeldenummer: PCT/EP2008/007888
(87) Internationale Veröffentlichungsnummer: WO 2009/049743

(56) Entgegenhaltungen:
- WO-A-2005/034950
- WO-A-2005/058315
- WO-A-2006/125531

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der TGF-beta-Rezeptorkinasen, eine Rolle spielt, ferner pharmazeutische Zusammensetzungen, die diese Verbindungen enthalten, sowie die Verwendung der Verbindungen zur Behandlung kinasebedingter Krankheiten.

Transforming growth factor beta ist der Prototyp der TGF-beta Superfamilie, einer Familie von hoch konservierten, pleiotrophen Wachstumsfaktoren, die sowohl während der Embryonalentwicklung als auch im adulten Organismus wichtige Funktionen ausüben. In Säugetieren wurden drei Isoformen von TGF-beta (TGF-beta 1, 2 und 3) identifiziert, wobei TGF-beta 1, die häufigste Isoform darstellt (Kingsley (1994) Genes Dev 8:133-146). TGF-beta 3 wird z.B. nur in mesenchymalen Zellen exprimiert, wohingegen TGF-beta 1 in mesenchmalen und epithelialen Zellen gefunden wird. TGF-beta wird als Präproprotein synthetisiert und in inaktiver Form in die extrazelluläre Matrix abgegeben (Derynck (1985) Nature 316: 701-705; Bottinger (1996) PNAS 93: 5877-5882). Neben der abgespaltenen Proregion, die auch als Latency Associated Peptide (LAP) bezeichnet wird und mit der reifen Region assoziiert bleibt, kann auch eines der 4 Isoformen der Latent TGF-beta Binding Proteins (LTBP 1-4) an TGF-beta gebunden sein (Gentry (1988) Mol Cell Biol 8: 4162-4168, Munger (1997) Kindey Int 51: 1376-1382). Die für die Entfaltung der biologischen Wirkung von TGF-beta notwendige Aktivierung des inaktiven Komplexes ist noch nicht vollständig geklärt. Allerdings ist mit Sicherheit eine proteolytische Prozessierung z.B. durch Plasmin, Plasma Transglutaminase oder Thrombospondin notwendig (Munger (1997) Kindey Int 51: 1376-1382). Der aktivierte Ligand TGF-beta vermittelt seine biologische Wirkung über drei membranständige TGF-beta Rezeptoren, die ubiquitär exprimierten Typ I und Typ II Rezeptoren und den Typ III Rezeptoren Betaglycan und Endoglin, wobei letzterer nur in Endothelzellen exprimiert wird (Gougos (1990) J Biol Chem 264: 8361-8364, Loeps-Casillas (1994) J Cell Biol 124:557-568). Beide Typ III TGF-beta Rezeptoren besitzen keine intrazelluläre Kinasedomäne, die eine Signalweiterleitung in die Zelle ermöglicht. Da die Typ III TGF-beta Rezeptoren alle drei TGF-beta Isoformen mit hoher Affinität binden und auch Typ II TGF-beta Rezeptor eine höhere Affinität für an Typ III Rezeptor gebundenen Liganden besitzt, besteht die biologische Funktion vermutlich in der Regulation der Verfügbarkeit der Liganden für Typ I und Typ II TGF-beta Rezeptoren (Lastres (1996) J Cell Biol 133:1109-1121; Lopes-Casillas (1993) Cell 73: 1435-1344). Die strukturell eng verwandten Typ I und Typ II Rezeptoren besitzen im zytoplasmatischen Bereich eine Serin/Threonin-Kinasedomäne, die für die Signalweiterleitung verantwortlich ist. Typ II TGF-beta Rezeptor bindet TGF-beta, woraufhin der Typ I TGF-beta Rezeptor zu diesem signalweiterleitenden Komplex rekrutiert wird. Die Serin/Threonin Kinasedomäne des Typ II Rezeptors ist konstitutiv aktiv und kann in diesem Komplex Serylreste in der sogenannten GS-Domäne des Typ I Rezeptors phosphorylieren. Diese Phosphorylierung aktiviert die Kinase des Typ I Rezeptors, die nun ihrerseits intrazelluläre Signalmediatoren, die SMAD Proteine phosphorylieren kann und damit die intrazelluläre Signalweiterleitung initiiert (zusammengefasst in Derynck (1997) Biochim Biophys Acta 1333: F105-F150).

Die Proteine der SMAD-Familie dienen als Substrate für alle TGF-beta Familien Rezeptor Kinasen. Bisher wurden 8 SMAD Proteine identifiziert, die sich in 3 Gruppen aufteilen: (1) Rezeptor-assozierte SMADs (R-SMADs) sind direkte Substrate der TGF-β Rezeptor Kinasen (SMAD1, 2, 3, 5, 8); (2) Co-SMADs, die mit den R-Smads während der Signalkaskade assoziieren (SMAD4); and (3) inhibitorische SMADs (SMAD6, 7), die die Aktivität der oben genannten SMAD Proteine hemmen. Von den verschiedenen R-SMADs, sind SMAD2 and SMAD3 die TGF-beta spezifischen Signalmediatoren. In der TGF-beta Signalkaskade werden also SMAD2/SMAD3 vom Typ I TGF-beta Rezeptor phosphoryliert, wodurch sie mit SMAD4 assoziieren können. Der entstandene Komplex aus SMAD2/SMAD3 und SMAD4 kann nun in den Zellkern translokalisert werden und dort direkt oder über andere Proteine die Transkription der TGF-beta regulierten Gene initiieren (zusammengefasst in Itoh (2000) Eur J Biochem 267: 6954-6967; Shi (2003) Cell 113:685-700).

Das Spektrum der Funktionen von TGF-beta ist breitgefächert und abhängig von Zellart und Differenzierungsstatus (Roberts (1990) Handbook of Experimental Pharmacology: 419-472). Zu den zellulären Funktionen, die von TGF-beta beeinflusst werden, gehören: Apoptose, Proliferation, Differenzierung, Mobilität und Zelladhäsion. Dementsprechend spielt TGF-beta eine wichtige Rolle in den verschiedensten biologischen Prozessen. Während der Embryonalentwicklung wird es an Orten der Morphogenese und insbesondere an Stellen mit epithelialermesenchymaler Interaktion exprimiert und induziert dort wichtige Differenzierungsprozesse (Pelton (1991) J Cell Biol 115:1091-1105). Eine Schlüsselfunktion übt TGF-beta auch bei der Selbsterneuerung und Aufrechterhaltung eines undifferenzierten Zustandes von Stammzellen aus (Mishra (2005) Science 310: 68-71). Zudem erfüllt TGF-beta auch in der Regulation des Immunsystems wichtige Funktionen. Es wirkt im allgemeinen immunsuppressiv, da es u.a. die Proliferation von Lymphozyten hemmt und die Aktivität von Gewebsmakrophagen einschränkt. TGF-beta lässt so inflammatorische Reaktionen wieder abklingen und hilft so überschießende Immunreaktionen zu vermeiden (Bogdan (1993) Ann NY Acad Sci 685: 713-739, zusammengefasst in Letterio (1998) Annu Rev Immunol 16: 137-161). Eine andere Funktion von TGF-beta ist die Regulation der Zellproliferation. TGF-beta hemmt das Wachstum von Zellen endothelialer, epithelialer und hämatopoetischer Herkunft, fördert aber das Wachstum von Zellen mesenchymalen Ursprungs (Tucker (1984) Science 226:705-707, Shipley (1986) Cancer Res 46:2068-2071, Shipley (1985) PNAS 82: 4147-4151). Eine weitere wichtige Funktion von TGF-beta ist die Regulation zellulärer Adhäsion und von Zell-Zell-Interaktionen. TGF-beta fördert den Aufbau der extrazellulären Matrix durch die Induktion von Proteinen der extrazellulären Matrix, wie z.B. Fibronectin und Kollagen. Zusätzlich reduziert TGF-beta die Expression von matrixdegradierenden Metalloproteasen und Inhibitoren der Metalloproteasen (Roberts (1990) Ann NY Acad Sci 580: 225-232; Ignotz (1986) J Biol Chem 261: 4337-4345; Overall (1989) J Biol Chem 264: 1860-1869); Edwards (1987) EMBO J 6: 1899-1904).

Das breite Wirkungsspektrum von TGF-beta impliziert, dass TGF-beta eine wichtige Rolle bei vielen physiologischen Gegebenheiten, wie der Wundheilung und bei pathologischen Prozessen, wie Krebs und Fibrose, spielt.

TGF-beta ist eines der Schlüssel-Wachstumsfaktoren bei der Wundheilung (zusammengefasst in O'Kane (1997) Int J Biochem Cell Biol 29: 79-89). Während der Granulationsphase wird TGF-beta an der Verletzungsstelle aus Blutplättchen freigegeben. TGF-beta reguliert sodann seine eigene Produktion in Makrophagen und induziert die Sekretion von anderen Wachstumsfaktoren z.B. durch Monozyten. Die wichtigsten Funktionen während der Wundheilung beinhalten die Stimulierung der Chemotaxis von inflammatorischen Zellen, die Synthese von extrazellulärer Matrix und die Regulation der Proliferation, Differenzierung und Genexpression aller wichtigen am Wundheilungsprozess beteiligten Zelltypen.

Unter pathologischen Bedingungen können diese TGF-beta vermittelte Effekte, insbesondere die Regulation der Produktion von extrazellulärer Matrix (ECM) zur Fibrose bzw. in der Haut zu Narben führen (Border (1994) N Engl J Med 331:1286-1292).

Für die fibrotischen Erkrankungen, diabetische Nephropathie und Glomeronephritis konnte nachgewiesen werden, dass TGF-beta renale Zellhypertrophie und die pathogene Akkumulation der extrazellulären Matrix fördert. Die Unterbrechung des TGF-beta Signalweges durch eine Behandlung mit anti-TGF-beta Antikörpern verhindert die Expansion der mesangialen Matrix, die progressive Abnahme der Nierenfunktion und reduziert etablierte Lesionen der diabetischen Glomerulopathie in diabetischen Tieren (Border (1990) 346: 371-374, Yu (2004) Kindney Int 66: 1774-1784, Fukasawah (2004) Kindney Int 65: 63-74, Sharma (1996) Diabetes 45: 522-530).

Auch bei der Leberfibrose spielt TGF-beta eine wichtige Rolle. Die für die Entwicklung der Leberfibrose wesentliche Aktivierung der hepatischen Sternzellen (engl. hepatic stellate cell) zu Myofibroblasten, den Hauptproduzent der extrazellulären Matrix im Rahmen der Entwicklung einer Leberzirrhose, wird von TGF-beta stimuliert. Auch hier konnte gezeigt werden, dass die Unterbrechung des TGF-beta Signalweges Fibrose in experimentellen Modellen reduziert (Yata (2002) Hepatology 35:1022-1030; Arias (2003) BMC Gastroenterol 3:29)

Eine Schlüsselfunktion nimmt TGF-beta auch bei der Krebsentstehung ein (zusammengefasst in Derynck (2001) Nature Genetics: 29: 117-129; Elliott (2005) J Clin Onc 23: 2078-2093). In frühen Stadien der Krebsentwicklung wirkt TGF-beta der Krebsentstehung entgegen. Diese tumorsupprimierende Wirkung beruht hauptsächlich auf der Fähigkeit von TGF-beta die Teilung von epithelialen Zellen zu inhibieren. Im Gegensatz dazu fördert TGF-beta Krebswachstum und Metastasenbildung in späten Tumorstadien. Dies lässt sich darauf zurückführen, dass die meisten epithelialen Tumoren eine Resistenz gegenüber der wachstumshemmenden Wirkung von TGF-beta entwickeln und TGF-beta gleichzeitig über andere Mechanismen das Wachstum der Krebszellen unterstützt. Zu diesen Mechanismen gehört die Förderung der Angiogenese, die immunsuppressive Wirkung, die Tumorzellen bei der Umgehung der Kontrollfunktion des Immunsystems (engl. immunosurveillance) unterstützt und die Förderung von Invasivität und Metastasenbildung. Die Ausbildung eines invasiven Phänotyps der Tumorzellen ist eine Hauptvoraussetzung für die Metastasenbildung. TGF-beta fördert diesen Prozess durch seine Fähigkeit die zelluläre Adhäsion, Motilität und die Ausbildung der extrazellulären Matrix zu regulieren. Weiterhin induziert TGF-beta die Umwandlung von einem epithelialen Phänotyp der Zelle zum invasiven mesenchymalen Phänotyp (engl. Epitheliale Mesenchymale Transition = EMT). Die wichtige Rolle, die TGF-beta bei der Förderung des Krebswachstums spielt, demonstrieren auch Untersuchungen, die eine Korrelation zwischen einer starken TGF-beta Expression und einer schlechten Prognose aufzeigen. Erhöhte TGF-beta Level wurden u.a. in Patienten mit Prostata-, Brust-, Darm- und Lungenkrebs gefunden (Wikström (1998) Prostate 37: 19-29; Hasegawa (2001) Cancer 91: 964-971; Friedman (1995), Cancer Epidemiol Biomarkers Prev. 4:549-54).

Aufgrund der oben beschriebenen krebsfördernden Wirkungen von TGF-beta bietet sich die Hemmung des TGF-beta Signalweges, z.B. über die Hemmung des TGF-beta Typ I Rezeptors als therapeutisches Konzept an. In zahlreichen präklinischen Versuchen konnte gezeigt werden, dass in der Tat die Unterbrechung des TGF-beta Signalweges das Krebswachstum hemmt. So reduziert die Behandlung mit löslichem TGF-beta Typ II Rezeptor die Bildung von Metastasen in transgenen Mäusen, die im Laufe der Zeit invasiven Brustkrebs entwickeln (Muraoka (2002) J Clin Invest 109: 1551-1559, Yang (2002) J Clin Invest 109: 1607-1615). Tumorzellinien, die einen defekten TGF-beta Typ II Rezeptor exprimieren, zeigen verringertes Tumor- und Metastasenwachstum (Oft (1998) Curr Biol 8: 1243-1252, McEachern (2001) Int J Cancer 91:76-82, Yin (1999) Jelin Invest 103: 197-206).

Zustände, "die durch eine gesteigerte TGF-β-Aktivität gekennzeichnet sind", umfassen solche Zustände, wobei die TGF-β-Synthese so stimuliert ist, dass das TGF-β in erhöhten Spiegeln vorhanden ist, oder wobei das latente TGF-β-Protein unerwünscht aktiviert oder in das aktive TGF-β-Protein umgewandelt ist oder wobei die TGF-β-Rezeptoren hochreguliert sind oder wobei das TGF-β-Protein eine gesteigerte Bindung an Zellen oder an die extrazelluläre Matrix am Krankheitsherd aufweist. Somit betrifft in jedem Fall "gesteigerte Aktivität" einen beliebigen Zustand, bei dem die biologische Aktivität von TGF-β unabhängig von der Ursache unerwünscht hoch ist.

Eine Reihe von Erkrankungen wurden mit der Überproduktion von TGF-β1 in Zusammenhang gebracht. Inhibitoren des intrazellulären TGF-β-Signalwegs sind geeignete Behandlungen für fibroproliferative Erkrankungen. Fibroproliferative Erkrankungen umfassen spezifisch Nierenstörungen, die mit einer unregulierten TGF-β-Aktivität einhergehen, und starke Fibrose, einschließlich Glomerulonephritis (GN), wie mesangiale proliferative GN, Immun-GN und Halbmond-GN. Andere Nierenzustände umfassen diabetische Nephropathie, renale interstitielle Fibrose, renale Fibrose bei Transplantat-Patienten, die Cyclosporin erhalten, und mit HIV einhergehende Nephropathie. Collagen-Gefäßstörungen umfassen progressive systemische Sklerose, Polymyositis, Sklerodermie, Dermatomyositis, eosinophile Fascitis, Morphea oder solche Störungen, die mit dem Vorkommen des Raynaud-Syndroms einhergehen. Lungenfibrosen, die durch eine übermäßige TGF-β-Aktivität verursacht werden, umfassen das Atemstörungssyndrom bei Erwachsenen, idiopathische Lungenfibrose und interstitielle Lungenfibrose, die oft mit Autoimmunstörungen einhergeht, wie systemischer Lupus erythematodes und Sklerodermie, chemischer Kontakt oder Allergien. Eine weitere Autoimmunstörung, die mit fibroproliferativen Eigenschaften einhergeht, ist rheumatoide Arthritis.

Augenerkrankungen, die mit einem fibroproliferativen Zustand einhergehen, umfassen eine proliferative Vitreoretinopathie, die bei einer Wiederbefestigungsoperation der Retina vorkommt, Katarakt-Extraktion mit einer intraokularen Linsenimplantation, und Post-Glaukom-Drainagenoperation, und gehen mit einer TGF-β1-Überproduktion einher.

Fibrose-Erkrankungen, die mit einer TGF-β1-Überproduktion einhergehen, können in chronische Zustände, wie die Fibrose der Niere, Lunge und Leber, und akutere Zustände, wie Hautvernarbung und Restenose, unterteilt werden (Chamberlain, J. Cardiovascular Drug Reviews, 19(4): 329-344). Die Synthese und die Sekretion von TGF-β1 durch Tumorzellen können ebenfalls zur Immunsuppression führen, wie es bei Patienten mit aggressivem Gehirn oder Brusttumoren beobachtet wird (Arteaga, et al. (1993) J. Clin. Invest. 92: 2569-2576). Der Verlauf der Leishmania-Infektion bei Mäusen wird durch TGF-β1 drastisch verändert (Barral-Netto, et al. (1992) Science 257: 545-547). TGF-β1 verschlechterte die Krankheit, wohingegen TGF-β1-Antikörper den Fortschritt der Erkrankung in genetisch anfälligen Mäusen aufhielten. Genetisch resistente Mäuse wurden bei der Verabreichung von TGF-β1 gegenüber Leishmania-Infektion anfällig.

Die tiefreichenden Wirkungen auf die Ablagerung der extrazellulären Matrix wurden im Überblick dargestellt (Rocco und Ziyadeh (1991) in Contemporary Issues in Nephrology v.23, Hormones, autocoids and the kidney, Hrsg. Jay Stein, Churchill Livingston, New York S. 391-410; Roberts, et al. (1988) Rec. Prog. Hormone Res. 44: 157-197) und umfassen die Stimulation der Synthese und die Hemmung des Abbaus der extrazellulären Matrixkomponenten. Da die Struktur- und Filtrationseigenschaften des Glomerulus zum Großteil durch die extrazelluläre Matrix-Zusammensetzung des Mesangiums und der glomerulären Membran bestimmt werden, ist es nicht überraschend, dass TGF-βl tiefreichende Wirkungen auf die Niere hat. Die Anreicherung der mesangialen Matrix bei der proliferativen Glomerulonephritis (Border, et al., (1990) Kidney Int. 37: 689-695) und der diabetischen Nephropathie (Mauer, et al. (1984) J. Clin. Invest. 74: 1143-1155) sind klare und dominante pathologische Merkmale der Erkrankungen. Die TGF-β1-Spiegel sind bei der diabetischen Glomerulosklerose beim Menschen (fortgeschrittene Neuropathie) erhöht (Yamamoto, et al. (1993) Proc. Natl. Acad. Sci. 90: 1814-1818). TGF-β1 ist ein wichtiger Vermittler bei der Genese der renaten Fibrose bei einer Reihe von Tiermodellen (Phan, et al. (1990) Kidney Int. 37: 426; Okuda, et al. (1990) J. Clin. Invest. 86: 453). Die Unterdrückung experimentell induzierter Glomerulonephritis in Ratten wurde durch Antiserum gegen TGF-β1 (Border, et al. (1990) Nature 346: 371) und durch ein extrazelluläres Matrixprotein, Decorin, das TGF-β1 binden kann, gezeigt (Border, et al. (1992) Nature 360: 361-363).

Zu viel TGF-β1 führt zur Bildung von Hautnarbengewebe. Das Neutralisieren von TGF-β1-Antikörpern, die in die Ränder heilender Wunden bei Ratten injiziert wurden, hemmte Befunden zufolge die Narbenbildung, ohne dass die Rate der Wundheilung oder die Zugfestigkeit der Wunde beeinträchtigt wurde (Shah, et al. (1992) Lancet 339: 213-214). Gleichzeitig war die Angiogenese niedriger, die Anzahl der Makrophagen und Monocyten in der Wunde geringer und das Ausmaß der disorganisierten Kollagenfaser-Ablagerung in dem Narbengewebe verringert.

TGF-β1 kann ein Faktor bei der progressiven Verdickung der Arterienwand sein, die von der Proliferation der glatten Muskelzellen und der Ablagerung von extrazellulärer Matrix in der Arterie nach der Ballongefäßplastik verursacht wird. Der Durchmesser der wiederverschlossenen Arterie kann durch diese Verdickung 90% reduziert sein, und da der Großteil der Reduktion des Durchmessers auf der extrazellulären Matrix und nicht auf den Körpern der glatten Muskelzellen beruht, kann man diese Gefäße wieder auf 50% öffnen, indem einfach die übermäßige Ablagerung der extrazellulären Matrix reduziert wird. Bei nicht verletzten Schweine-Arterien, die mit einem TGF-β1-Gen in vivo transfiziert wurden, ging die TGF-β1-Genexpression sowohl mit der Synthese der extrazellulärer Matrix als auch mit Hyperplasie einher (Nabel, et al. (1993) Proc. Natl. Acad. Sci USA 90: 10759-10763). Die durch TGF-β1 induzierte Hyperplasie war nicht so ausgiebig, wie diejenige, die mit PDGF-BB induziert wurde, jedoch war die extrazelluläre Matrix bei TGF-β1-Transfektanten ausgeprägter. Es gab keine Ablagerung extrazellulärer Matrix bei einer durch FGF-1 (einer sezemierten Form von FGF) induzierten Hyperplasie bei diesem Genübertragungsmodell beim Schwein (Nabel (1993) Nature 362: 844-846).

Es gibt verschiedene Arten von Krebs, wobei das vom Tumor erzeugte TGF-β1 schädlich sein kann. MATLyLu-Prostatakrebszellen bei der Ratte (Steiner und Barrack (1992) Mol. Endocrinol 6: 15-25) und MCF-7 Brustkrebszellen beim Menschen (Arteaga, et al. (1993) Cell Growth and Differ. 4: 193-201) wurden nach der Transfektion mit einem Vektor, der das Maus- TGF-β1 exprimierte, tumorigener und metastatischer. TGF-β1 ging mit Angiogenese, Metastase und schlechter Prognose bei Human-Prostata und fortgeschrittenem Darmkrebs einher (Wikstrom, P., et al. (1988) Prostate 37; 19-29; Saito, H., et al. (1999) Cancer 86: 1455-1462). Bei Brustkrebs geht eine schlechte Prognose mit erhöhtem TGF-β einher (Dickson, et al. (1987) Proc. Natl. Acad. Sci. USA 84: 837-841; Kasid, et al. (1987) Cancer Res. 47: 5733-5738; Daly, et al. (1990) J. Cell Biochem. 43: 199-211; Barrett-Lee, et al. (1990) Br. J. Cancer 61: 612-617; King, et al (1989) J. Steroid Biochem. 34: 133-138; Welch, et al (1990) Proc. Natl. Acad. Sci USA 87: 7678-7682; Walker et al. (1992) Eur. J. Cancer 238: 641-644), und die Induktion von TGF-β1 durch Tamoxifen-Behandlung (Butta, et al. (1992) Cancer Res. 52: 4261-4264) ging mit einem Versagen der Tamoxifen-Behandlung bei Brustkrebs einher (Thompson, et al. (1991) Br. J. Cancer 63: 609-614). Anti-TGF-β1-Antikörper hemmen das Wachstum von MDA-231-Human-Brustkrebszellen in athymischen Mäusen (Arteaga, et al. (1993) J. Clin. Invest. 92: 2569-2576), eine Behandlung, die mit einem Anstieg der natürlichen Killerzellaktivität in der Milz korreliert ist. CHO-Zellen, die mit latentem TGF-β1 transfiziert sind, zeigten ebenfalls gesenkte NK-Aktivität und gesteigertes Tumorwachstum in Nacktmäusen (Wallick, et al. (1990) J. Exp. Med. 172: 177-1784). Somit kann das durch Brusttumore sezernierte TGF-β eine endokrine Immunsuppression verursachen. Hohe Plasmakonzentrationen von TGF-β1 zeigen eine schlechte Prognose für Patienten mit fortgeschrittenem Brustkrebs (Anscher, et al. (1993) N. Engl. J. Med. 328: 1592-1598). Patienten mit hohem zirkulierendem TGF-β vor der Hochdosis-Chemotherapie und autologer Knochenmarktransplantation haben ein hohes Risiko für eine hepatische venookklusive Erkrankung (15-50% sämtlicher Patienten mit einer Mortalitätsrate bis zu 50%) und eine idiopathische interstitielle Pneumonitis (40 bis 60% sämtlicher Patienten). Die Bedeutung dieser Befunde ist, dass 1) erhöhte Plasmaspiegel von TGF-β1 zur Identifikation von Risikopatienten verwendet werden können, und dass 2) eine Reduktion von TGF-β1 die Morbidität und Mortalität dieser üblichen Behandlungen für Brustkrebspatienten senken kann.

Viele maligne Zellen sezernieren transformierenden Wachstumsfaktor β (TGF-β), ein leistungsfähiges Immunsuppressivum, was nahe legt, dass die TGF-β-Produktion einen signifikanten Tumor-Escape-Mechanismus vor der Wirts-Immunüberwachung darstellen kann. Die Errichtung einer Leukocyten-Subpopulation mit unterbrochenem TGF-β-Signalweg in dem tumortragenden Wirt bietet eine leistungsfähige Maßnahme zur Immuntherapie von Krebs. Ein transgenes Tiermodell mit unterbrochenem TGF-β-Signalweg in T-Zellen kann einen normal letalen TGF-β-überexprimierenden Lymphom-Tumor, EL4, auslöschen (Gorelik und Flavell, (2001) Nature Medicine 7(10): 1118-1122). Das Herunterregulieren der TGF-β-Sekretion in Tumorzellen führt zur Wederherstellung der Immunogenität im Wirt, wohingegen die T-Zell-Unempfindlichkeit gegenüber TGF-β zu einer beschleunigten Differenzierung und Autoimmunität führt, deren Elemente erforderlich sein können, um die Self-Antigen-exprimierenden Tumore in einem tolerant gemachten Wirt zu bekämpfen. Die immunsuppressiven Wirkungen von TGF-β sind auch bei einer Subpopulation von HIV-Patienten mit einer niedrigeren Immunreaktion als vorhergesagt auf der Basis ihrer CD4/CD8-T-Zellzahlen beteiligt (Garba, et al., J. Immunology (2002) 168: 2247-2254). Ein TGF-β-neutralisierender Antikörper konnte die Wirkung in der Kultur umkehren, was anzeigt, dass sich TGF-β-Signalweg-Inhibitoren bei der Umkehr der Immunsuppression, die bei diesem Anteil von HIV-Patienten vorliegt, eignen können.

Während der frühesten Stufen der Karzinogenese kann TGF-β1 als leistungsfähiger Tumorsuppressor wirken und kann die Wirkungen einiger chemopräventiver Mittel vermitteln. Bei einem gewissen Punkt während der Entwicklung und des Verlaufs maligner Neoplasmen scheinen sich Tumorzellen von der TGF-β-abhängigen Wachstumshemmung parallel zum Erscheinen von biologisch aktivem TGF-β in der Mikroumgebung zu entziehen. Die doppelte Tumorsuppressions- bzw. Tumorförderungs-Rolle von TGF-β wurde am deutlichsten in einem transgenen System gezeigt, das TGF-β in Keratinocyten überexprimiert. Transgene waren zwar gegenüber der Bildung gutartiger Hautläsionen resistenter, jedoch war die Rate der Metastasen-Konversion bei den Transgenen drastisch erhöht (Cui, et al. (1996) Cell 86(4): 531-42). Die Produktion von TGF-β1 durch maligne Zellen in primären Tumoren scheint mit fortschreitenden Stufen der Tumorprogression zu steigen. Untersuchungen bei vielen Hauptepithelkrebsarten legen nahe, dass die erhöhte Produktion von TGF-β durch Krebs beim Mensch als relativ spätes Ereignis während der Tumorprogression eintritt. Zudem verhilft dieses tumorassoziierte TGF-β den Tumorzellen zu einem selektiven Vorteil und fördert die Tumorprogression. Die Wirkungen von TGF-β auf Zell-Zell- und Zell-Stroma-Wechselwirkungen führt zu einer größeren Neigung für die Invasion und Metastase. Tumorassozüertes TGF-β kann es Tumorzellen ermöglichen, sich der Immunüberwachung zu entziehen, da es ein leistungsfähiger Inhibitor der klonalen Expansion aktivierter Lymphocyten ist. Es wurde auch gezeigt, dass TGF-β die Produktion von Angiostatin hemmt. Krebstherapie-Modalitäten, wie Strahlungstherapie und Chemotherapie, induzieren die Produktion von aktiviertem TGF-β im Tumor, wodurch das Auswachsen maligner Zellen selektiert wird, die gegenüber TGF-β-wachstumsinhibitorischen Wirkungen resistent sind. Somit steigern diese Antikrebs-Behandlungen die Gefahr und beschleunigen die Entwicklung von Tumoren mit gesteigertem Wachstum und Invasionsvermögen. In dieser Situation können Mittel, die die TGF-β-vermittelte Signaltransduktion ansteuern, eine sehr effiziente Therapiestrategie sein. Es wurde gezeigt, dass die Resistenz der Tumorzellen gegenüber TGF-β einen Großteil der cytotoxischen Wirkungen der Strahlungstherapie und Chemotherapie unwirksam macht, und die behandlungsabhängige Aktivierung von TGF-β im Stroma kann sogar schädlich sein, da es die Mikroumgebung gegenüber der Tumorprogression leitfähiger macht und zur Gewebeschädigung beiträgt, was zu Fibrose führt. Die Entwicklung von TGF-β-Signaltransduktionsinhibitoren hat wahrscheinlich einen Vorteil für die Behandlung von fortgeschrittenem Krebs allein und in Kombination mit anderen Therapien.

Die Verbindungen eignen sich zur Behandlung von Krebs und anderen Erkrankungszuständen, die durch TGF-β beeinflusst werden, durch Hemmen von TGF-β in einem Patienten, der dieses benötigt, indem dem Patient die Verbindung(en) verabreicht wird bzw. werden. TGF-β ist auch geeignet gegen Atherosklerose- (T.A. McCaffrey: TGF-βs and TGF-β Receptors in Atherosclerosis: Cytokine and Growth Factor Reviews 2000, 11, 103-114) und Alzheimer-Erkrankungen (Masliah, E.; Ho, G.; Wyss-Coray, T.: Functional Role of TGF-β in Alzheimer's Disease Microvascular Injury: Lessons from Transgenic Mice: Neurochemistry International 2001, 39, 393-400).

Es wurde gefunden, daß die erfindungsgemäßen Verbindungen und ihre Salze bei guter Verträglichkeit sehr wertvolle pharmakologische Eigenschaften besitzen.
Insbesondere zeigen sie TGF□-Rezeptor I-Kinase inhibierende Eigenschaften.

Die erfindungsgemäßen Verbindungen zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des TGF□-Signalwegs.

Ein weiterer Gegenstand der vorliegenden Erfindung ist die Verwendung einer oder mehrerer erfindungsgemäßer Verbindungen bei der Behandlung und/oder Prophylaxe von Erkrankungen, bevorzugt den hier beschriebenen Erkrankungen, die durch eine gesteigerte TGF□-Aktivität verursacht, vermittelt und/oder propagiert werden.

Gegenstand der vorliegenden Erfindung sind deshalb erfindungsgemäße Verbindungen als Arzneimittel und/oder Arzneimitteiwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit □ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

### STAND DER TECHNIK

5-Cyano-thienopyridine, die in 4-Stellung mit einem Phenylring substituiert sind, sind bekannt aus WO 2006/125531 A2.
WO 2005/034950 A1 beschreibt weitere 5-Cyano-thienopyridine, die als Inhibitoren des HSP90 (Heat Shock Protein 90) wirken.
WO 2005/058315 A1 beschreibt die Verwendung von 5-Cyanothienopyridinen zur Behandlung der Hepatitis B.

### ZUSAMMENFASSUNG DER ERFINDUNG

Die Erfindung betrifft Verbindungen der Formel I 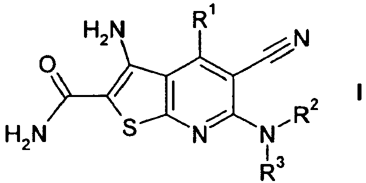 worin
- R¹: Het
- R², R³: jeweils unabhängig voneinander H, A, AlkNH₂, AlkNHA, AlkNAA', AlkNHSO₂A, AlkOH, AlkOA, AlkCyc, AlkCycAlkOH, AlkCycAlkOA, AlkCycAlkCOOA, AlkCycAlkCOOH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ oder AlkAr, wobei einer der Substituenten R2 und R3 ≠ H ist,
- R² und R³: zusammen auch eine Alkylenkette mit 1 bis 6 C-Atomen sein kann, worin eine oder zwei nicht benachbarte CH₂-Gruppen durch N- und/oder O-Atome und/oder worin 1 bis 6 H-Atome durch A, OH, OA, (CH₂)ₙHet¹, SO₂A und/oder Hal ersetzt sein können,
- Alk: Alkylen mit 1 bis 6 C-Atomen, worin 1 bis 4 H-Atome durch F, Cl und/oder Br ersetzt sein können,
- Cyc: Cycloalkyl mit 3 bis 7 C-Atomen, worin 1 bis 4 H-Atome durch A, Hal, OH und/oder OA ersetzt sein können,
- Het: Benzofuranyl, das ein- oder zweifach durch A substituiert sein kann,
- Het¹: einen einkernigen gesättigten, ungesättigten oder aromatischen Heterocyclus mit 1 bis 4 N-, O- und/oder S-Atomen, der ein-, zwei- oder dreifach durch A, OH, OA, Hal, SO₂A und/oder =O (Carbonylsauerstoff) substituiert sein kann,
- Ar: Phenyl, das unsubstituiert oder ein-, zwei- oder dreifach durch A, OH, OA, Hal, SO₂NH₂, SO₂NA und/oder SO₂NAA' substituiert ist,
- A, A': jeweils unabhängig voneinander unverzweigtes oder verzweigtes Alkyl mit 1-10 C-Atomen, worin 1-5 H-Atome durch F, Cl und/oder Br ersetzt sein können,
- Hal: F, Cl, Br oder I,
- m: 1, 2, 3, 4,
- n: 0, 1, 2, 3, 4
bedeuten,
sowie ihre pharmazeutisch verwendbaren Salze, Solvate, Tautomere und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter Prodrug-Derivaten versteht man mit z. B. Alkyl- oder Acylgruppen, Zuckern oder Oligopeptiden abgewandelte erfindungsgemäße Verbindungen, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1 bis 7 sowie ihrer pharmazeutisch verwendbaren Solvate, Salze, Tautomere und Stereoisomere, dadurch gekennzeichnet, dass man
a) zur Herstellung einer Verbindung der Formel I worin R², R³ = H sind,
   eine Verbindung der Formel II 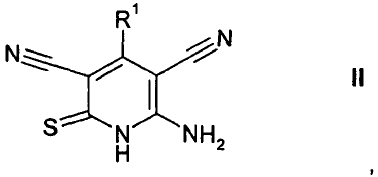 worin R¹ die in Anspruch 1 angegebene Bedeutung hat,
   mit einer Verbindung der Formel III

   H₂N-CO-CH₂-Z III

   worin
   - Z: Cl, Br, I oder eine freie oder reaktionsfähige funktionell abgewandelte OH-Gruppe bedeutet,
   zu einer Verbindung der Formel IV 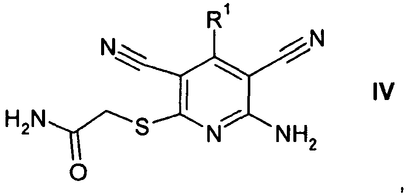 worin R¹ die in Anspruch 1 angegebene Bedeutung hat,
   umsetzt,
   und die erhaltene Verbindung der Formel IV anschließend zur Verbindung der Formel I zyklisiert,
   oder
b) zur Herstellung der Verbindung der Formel 1, worin mindestens einer der beiden Reste R², R³ ≠ H ist,
   in einer Verbindung der Formel IV die freie Aminogruppe gegen Hal austauscht,
   wobei eine Verbindung der Formel V 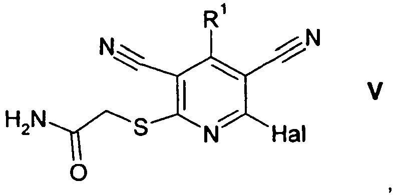 worin R¹ die in Anspruch 1 angegebene Bedeutung hat,
   erhalten wird,
   die Verbindung der Formel V mit einer Verbindung der Formel VI 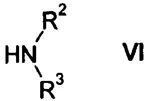 worin R² und R³ die in Anspruch 1 angegebenen Bedeutungen haben, jedoch mindestens einer der beiden Reste R², R³ ≠ H ist,
   zu einer Verbindung der Formel VII 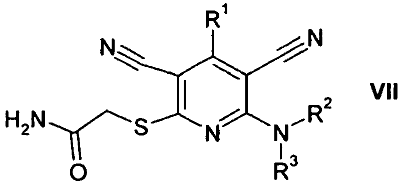 worin R¹, R², R³ die in Anspruch 1 angegebenen Bedeutungen haben, jedoch mindestens einer der beiden Reste R², R³ ≠ H ist,
   umsetzt
   und die erhaltene Verbindung der Formel VII anschließend zur Verbindung der Formel I zyklisiert,
   und/oder
c) eine Base oder Säure der Formel I in eines ihrer Salze umwandelt.

Gegenstand der Erfindung sind auch die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die erfindungsgemäßen Verbindungen der Formel I können auch in tautomeren Formen vorliegen. Formel I umfaßt alle diese tautomeren Formen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.
Vor- und nachstehend haben die Reste R¹, R² und R³ die bei der Formel I angegebenen Bedeutungen, falls nicht ausdrücklich etwas anderes angegeben ist.

A A' bedeuten unabhängig voneinander Alkyl, ist unverzweigt (linear) oder verzweigt, und hat 1, 2, 3, 4, 5, 6, 7, 8, 9 oder 10 C-Atome. A bedeutet besonders bevorzugt Methyl, Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-oder 1,2, 2-Trimethylpropyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl, ferner auch Fluormethyl, Difluormethyl oder Brommethyl.

Cyc ist, unabhängig weiterer Substitutionen, Cycloalkyl und bedeutet vorzugsweise Cyclopropyl, Cyclobutyl, Cylopentyl, Cyclohexyl oder Cycloheptyl, insbesondere Cyclopropyl und Cyclobutyl.

Alk bedeutet C₁-C₁₀ Alkylen, vorzugsweise Methylen, Ethylen, Propylen, Butylen, Pentylen, Hexylen, Heptylen, Octylen, Nonylen oder Decylen, Isopropylen, Isobutylen, sek.-Butylen, 1-, 2- oder 3-Methylbutylen, 1,1- , 1,2- oder 2,2-Dimethylpropylen, 1-Ethylpropylen, 1- , 2- , 3- oder 4-Methylpentylen, 1,1- , 1,2- , 1,3- , 2,2- , 2,3- oder 3,3-Dimethylbutylen, 1- oder 2-Ethylbutylen, 1-Ethyl-1-methylpropylen, 1-Ethyl-2-methylpropylen, 1,1,2- oder 1,2,2-Trimethylpropylen. Bevorzugt ist C₁-C₆ Alkylen, besonders bevorzugt Methylen, Ethylen, Propylen, Butylen, Pentylen oder Hexylen.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-Sulfonamidophenyl, o-, m- oder p-(N-Methyl-Sulfonamido)phenyl, o-, m- oder p-(N,N-Dimethyl-Sulfonamido)phenyl, o-, m- oder p-(N-Ethyl-N-Methyl-Sulfonamido)phenyl, o-, m- oder p-(N,N-Diethyl-Sulfonamido)phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl oder 2,5-Dimethyl-4-chlorphenyl.

Ar bedeutet vorzugsweise unsubstituiertes oder ein-, zwei- oder dreifach durch A, OH, OA, Hal, SO₂NH₂, SO₂NA und/oder SO₂NAA' substituiertes Phenyl. Besonders bevorzugt ist Ar Phenyl, das einfach durch SO₂NH₂, SO₂NA oder SO₂NAA' substituiert ist.

Het bedeutet, ungeachtetet weiterer Substitutionen, Benzofuran-3-, 4-, 5-oder 6-yl.

Het¹ bedeutet vorzugsweise einen einkernigen gesättigten oder aromatischen Heterocyclus mit 1 bis 2 N- und/oder O-Atomen, der ein- oder zweifach durch A, OH, OA, Hal, SO₂A und/oder =O (Carbonylsauerstoff) substituiert sein kann.

In einer weiteren Ausführungsform bedeutet Het¹, besonders bevorzugt unsubstituiertes oder ein- oder zweifach durch A, OH, OA, Hal, SO₂A und/oder =O (Carbonylsauerstoff) substituiertes Piperidin, Piperazin, Pyrrolidin, Morpholin, Furan, Tetrahydropyran, Pyridin, Pyrrol, Indol, Indazol, Isoxazol oder Imidazol, wobei A vorzugsweise Methyl, Ethyl, Propyl, Butyl, Pentyl, Hexyl, Isopropyl oder Trifluormethyl, Hal vorzugsweise F, Cl oder Br, OA vorzugsweise Methoxy, Ethoxy oder Propoxy bedeutet und in SO₂A als A bevorzugt Methyl, Ethyl, Propyl oder Butyl enthalten ist.
Ganz besonders bevorzugt ist unsubstituiertes oder ein- oder zweifach durch A, OH, OA, Hal, SO₂A und/oder =O (Carbonylsauerstoff) substituiertes Piperidin, Piperazin, Pyrrolidin, Morpholin, Furan, Tetrahydropyran, Tetrahydrofuran, Indazol, Isoxazol oder Imidazol, wobei A vorzugsweise Methyl, Ethyl, Propyl, Isopropyl, Butyl oder Trifluormethyl, Hal vorzugsweise F oder Cl, OA vorzugsweise Methoxy, Ethoxy oder Propoxy bedeutet und in SO₂A als A bevorzugt Methyl, Ethyl, Propyl oder Butyl enthalten ist.
Ar bedeutet Phenyl, das einfach durch SO₂NH₂, SO₂NA oder SO₂NAA' substituiert ist, wobei SO₂NH₂ besonders bevorzugt ist.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Im ausgedrückt werden, die der Formel I entsprechen und worin die nicht näher bezeichneten Reste die bei der Formel I angegebene Bedeutung haben, worin jedoch

| | | |
|---|---|---|
| in Ia | R² | H, A, AlkNH₂, AlkNHA, AlkNAA', AlkNHSO₂A, AlkOH, AlkOA, AlkCyc, AlkCycAlkOH, AlkCycAlkOA, AlkCycAlkC0OA, AlkCycAlkC0OH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ oder AlkAr und |
| | R³ | H bedeutet; |
| in Ib | R² und R³ | zusammen auch eine Alkylenkette mit 1 bis 5 C-Atomen sein kann, worin eine nicht benachbarte CH₂-Gruppe durch ein N- oder O-Atom und/oder worin 1 oder 2 H-Atom durch A, OH, OA, (CH₂)ₙHet¹, und/oder SO₂A ersetzt sein können, |
| In Ic | Alk | Methylen, Ethylen, Propylen, Butylen, Pentylen oder Hexylen bedeutet; |
| in Id | Cyc | Cyclopropan, Cyclobutan, Cyclopentan oder Cyclohexan, das unsubstituiert oder einfach durch OH oder OA substituiert sein kann; |
| in Ig | Het | Benzofuranyl, das ein- oder zweifach durch A substituiert sein kann; |
| in Ih | Het¹ | einen einkernigen gesättigten oder ungesättigten Heterocyclus mit 1 bis 3 N-, O- und/oder S-Atomen, der ein-, zwei- oder dreifach durch A, Hal, SO₂A und/oder =O (Carbonylsauerstoff) substituiert sein kann oder |
| in li | Het¹ | einen einkernigen gesättigten oder ungesättigten Heterocyclus mit 1 bis 2 N- und/oder O-Atomen, der ein- oder zweifach durch A und/oder =O (Carbonylsauerstoff) substituiert sein kann |
| in Ij | Het¹ | Piperazinyl, Morpholinyl, Piperidinyl, Pyrrolidinyl, Tetrahydropyranyl, Tetrahydrofuranyl, Imidazolyl, das unsubstituiert oder ein- oder zweifach durch A, und/oder =O (Carbonylsauerstoff) substituiert sein kann, |
| in Ik | Ar | Phenyl, das einfach durch SO₂NH₂, SO₂NA oder SO₂NAA' substituiert ist; |
| in II | A, A' | unverzweigtes oder verzweigtes Alkyl mit 1-6 C-Atomen, worin 1-5 H-Atome durch F und/oder Cl ersetzt sein können, |
| in Im | R¹ | Het |
| | R² | H, A, AlkNH₂, AlkNHA, AlkNAA', AlkNHSO₂A, AlkOH, AlkOA, AlkCyc, AlkCycAlkOH, AlkCycAlkOA, AlkCycAlkC0OA, AlkCycAlkCOOH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ oder AlkAr, |
| | R³ | H, |
| | R² und R³ | zusammen auch eine Alkylenkette mit 1 bis 5 C-Atomen sein kann, worin eine nicht benachbarte CH₂-Gruppe durch ein N- oder ein O-Atom und/oder worin 1 H-Atom durch A, OH, OA, (CH₂)ₙHet¹, oder SO₂A ersetzt sein können, |
| | Alk | Methylen, Ethylen, Propylen, Butylen, Pentylen oder Hexylen, |
| | Cyc | Cyclopropan, Cyclobutan, Cyclopentan oder Cyclohexan, das unsubstituiert oder einfach durch OH substituiert sein kann, |
| | Het | Benzofuranyl, das ein- oder zweifach durch A substituiert sein kann, |
| | Het¹ | einen einkernigen gesättigten Heterocyclus mit 1 bis 2 N- und/oder O-Atomen, der ein- oder zweifach durch A und/oder =O (Carbonylsauerstoff) substituiert sein kann, |
| | Ar | Phenyl, das einfach durch SO₂NH₂, SO₂NA oder SO₂NAA' substituiert ist, |
| | A, A' | unverzweigtes oder verzweigtes Alkyl mit 1-6 C-Atomen, worin 1-5 H-Atome durch F und/oder Cl ersetzt sein können, |
| | Hal | F, Cl, Br oder I, |
| | m | 1, 2, 3, 4, |
| n | | 0,1,2,3,4 |
| | | bedeuten |

sowie ihre pharmazeutisch verwendbaren Derivate, Solvate, Salze, Tautomere und Stereoisomere, einschließlich deren Mischungen in allen Verhältnissen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I, worin R² und R³ = H sind, können vorzugsweise erhalten werden, indem man eine Verbindung der Formel II mit einer Verbindung der Formel III umsetzt.
Die Verbindungen der Formel II und III sind in der Regel bekannt. Sind sie nicht bekannt, so können sie nach an sich bekannten Methoden hergestellt werden.

In den Verbindungen der Formel III bedeutet Z vorzugsweise Cl, Br, I oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy). Z bedeutet besonders bevorzugt Cl.

Die Umsetzung erfolgt nach Methoden, die dem Fachmann bekannt sind.

Die Umsetzung erfolgt vorzugsweise unter basischen Bedingungen. Als Basen eignen sich vorzugsweise Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin oder Diethanolamin.

Die Reaktion erfolgt in einem geeigneten inerten Lösungsmittel.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Als Lösungsmittel besonders bevorzugt ist z.B. Wasser und/oder Tetrahydrofuran.

Bei der Reaktion wird zunächst eine Verbindung der Formel IV gebildet, die anschließend zur Verbindung der Formel I zyklisiert. Die Verbindung der Formel IV kann als Zwischenprodukt isoliert werden und beispielsweise als Ausgangsverbindung für die Herstellung von Verbindungen der Formel I, worin mindestens einer der beiden Reste R², R³ ≠ H ist, verwendet werden.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 130°, insbesondere zwischen etwa 30° und etwa 125°.

Verbindungen der Formel I, worin mindestens einer der beiden Reste R², R³ ≠ H ist, können vorzugsweise erhalten werden, indem man in einer Verbindung der Formel IV zunächst die freie Aminogruppe gegen Hal austauscht, CSF CSF die entstehende Verbindung der Formel V mit einer Verbindung der Formel VI zu einer Verbindung der Formel VII umsetzt und diese anschließend zyklisiert.

Die Reaktion erfolgt vorzugsweise in inerten Lösungsmitteln wie oben beschrieben, besonders bevorzugt sind Aceton, Acetonitril und/oder Ethanol.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 130°, insbesondere zwischen etwa 30° und etwa 125°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet.

Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der Verbindungen der Formel I unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Efindung sind ferner Arzneimittel, enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,1 mg bis 3 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapsein eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Vernebiern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung der Formel I per se bestimmt werden. Es läßt sich annehmen, daß ähnliche Dosierungen für die Behandlung der anderen, obenerwähnten Krankheitszustände geeignet sind.

Gegenstand der Efindung sind Arzneimittel enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe und mindestens einen weiteren Arzneimittelwirkstoff.

Gegenstand der Erfindung ist ferner die Verwendung von Verbindungen der Formel I sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung und/oder Bekämpfung von Krebs, Tumorwachstum, Metastasenwachstum, wobei der Tumor ausgewählt ist aus der Gruppe der Tumoren des Plattenepithel, der Blasen, des Magens, der Nieren, von Kopf und Hals, des Ösophagus, des Gebärmutterhals, der Schilddrüse, des Darms, der Leber, des Gehirns, der Prostata, des Urogenitaltrakts, des lymphatischen Systems, des Magens, des Kehlkopfs, der Lunge, Lungenadenokarzinom, kleinzelliges Lungenkarzinom, Bauchspeicheldrüsenkrebs, Glioblastom, Kolonkarzinom, Brustkarzinom, Tumor des Blut- und Immunsystems, akute myelotische Leukämie, chronische myelotische Leukämie, akute lymphatische Leukämie, chronische lymphatische Leukämie.

Als weitere Arzneimittelwirkstoffe sind Chemotherapeutika bevorzugt, insbesondere solche, die Angiogenese hemmen und dadurch das Wachstum und die Verbreitung von Tumorzellen inhibieren; bevorzugt sind dabei VEGF-Rezeptorinhibitoren, beinhaltend Robozyme und Antisense, die auf VEGF-Rezeptoren gerichtet sind, sowie Angiostatin und Endostatin.

Beispiele antineoplastischer Agenzien, die in Kombination mit den erfindungsgemäßen Verbindungen verwendet werden können, beinhalten im allgemeinen alkylierende Agenzien, Antimetaboliten; Epidophyllotoxin; ein antineoplastisches Enzym; einen Topoisomerase-Inhibitor; Procarbazin; Mitoxantron oder Platin-Koordinationskomplexe.

Antineoplastische Agenzien sind vorzugsweise ausgewählt aus den folgenden Klassen:
Anthracycline, Vinca-Arzneistoffe, Mitomycine, Bleomycine, cytotoxische Nukleoside, Epothilone, Discodermolide, Pteridine, Diynene und Podophyllotoxine.
Besonders bevorzugt sind in den genanten Klassen z.B. Carminomycin, Daunorubicin, Aminopterin, Methotrexat, Methopterin, Dichlormethotrexat, Mitomycin C, Porfiromycin, 5-Fluoruracil, 5-Fluordeoxyuridin Monophosphat, Cytarabine, 5-Azacytidin, Thioguanin, Azathioprine, Adenosin, Pentostatin, Erythrohydroxynonyladenin, Cladribine, 6-Mercaptopurin, Gemcitabine, Cytosinarabinosid, Podophyllotoxin oder Podophyllotoxinderivate, wie z.B. Etoposide, Etoposide Phosphat oder Teniposide, Melphalan, Vinblastine, Vinorelbine, Vincristine, Leurosidine, Vindesine, Leurosine, Docetaxel und Paclitaxel. Andere bevorzugte antineoplastische Agenzien sind ausgewählt aus der Gruppe Discodermolide, Epothilone D, Estramustine, Carboplafin, Cisplatin, Oxaliplatin, Cyclophosphamid, Bleomycin, Gemcitabine, Ifosamide, Melphalan, Hexamethylmelamin, Thiotepa, Idatrexate, Trimetrexate, Dacarbazine, L-Asparaginase, Camptothecin, CPT-11, Topotecan, Arabinosyl-Cytosin, Bicalutamide, Flutamide, Leuprolide, Pyridobenzoindolderivate, Interferone und Interleukine.

Als weitere Arzneimittelwirkstoffe sind Antibiotica bevorzugt. Bevorzugte Antibiotica sind ausgewählt aus der Gruppe
Dactinomycin, Daunorubicin, Idarubicin, Epirubicin, Mitoxantrone, Bleomycin, Plicamycin, Mitomycin.

Als weitere Arzneimittelwirkstoffe sind Enzyminhibitoren bevorzugt. Bevorzugte Enzyminhibitoren sind ausgewählt aus der Gruppe der Histon-Deacetylierungs-Inhibitoren (z.B. suberoylanilide hydroxamic acid [SAHA]) und der Tyrosinkinase-Inhibitoren (z.B. ZD 1839 [Iressa]).

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Immunsuppressiva bevorzugt. Bevorzugte Immunsuppressiva sind ausgewählt aus der Gruppe Rapamycin, CCI-779 (Wyeth), RAD001 (Novartis), AP23573 (Ariad Pharmaceuticals).

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Die erfindungsgemäßen Verbindungen sowie ihre pharmazeutisch verwendbaren Salze, Solvate, Tautomeren und Stereoisomeren, einschließlich deren Mischungen in allen Verhältnissen, eignen sich als pharmazeutische Wirkstoffe für Säugetiere, insbesondere für den Menschen, zur Herstellung eines Arzneimittels zur Behandlung und/oder Bekämpfung von Krebs, Tumorwachstum, Metastasenwachstum, Fibrose, Restenose, HIV Infektion, Alzheimer, Atherosklerose und/oder zur Förderung der Wundheilung.

Gegenstand der Erfindung ist daher die Verwendung von Verbindungen der Formel I sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung und/oder Bekämpfung von Krebs, Tumorwachstum, Metastasenwachstum, Fibrose, Restenose, HIV Infektion, Alzheimer, Atherosklerose, und/oder zur Förderung der Wundheilung.

Insbesondere bevorzugt ist die Verwendung zur Behandlung einer Krankheit, wobei die Krankheit ein fester Tumor ist.
Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Gegenstand der Erfindung ist auch die Verwendung von Verbindungen gemäß Anspruch 1 und/oder ihrer physiologisch unbedenklichen Salze und Solvate zur Herstellung eines Arzneimittels zur Behandlung von festen Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).

Gegenstand der Erfindung ist daher auch die Verwendung von Verbindungen gemäß Anspruch 1 und/oder ihrer physiologisch unbedenklichen Salze und Solvate zur Herstellung eines Arzneimittels zur Behandlung von festen Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodutatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, lrinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylen-dioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### Zellulärer Assay zur Testung von TGF-beta-Rezeptor-I-Kinase-Inhibitoren

Als Beispiel wird die Fähigkeit der Inhibitoren zur Aufhebung der TGF-beta vermittelten Wachstumshemmung getestet.
Zellen der Lungenepithelzellinie Mv1 Lu werden in definierter Zelldichte in einer 96-well Mikrotiterplatte ausgesät und über 16 Stunden unter Standardbedingungen kultiviert. Anschließend wird das Medium mit Medium, das 0,5%FCS und 1 ng/ml TGF-beta enthält, ersetzt und die Testsubstanzen in definierten Konzentrationen, in der Regel in Form von Verdünnungsreihen mit 5-fach Schritten, zugegeben. Die Konzentration des Lösungsmittels DMSO liegt konstant bei 0,5%. Nach 48 Stunden erfolgt Kristallviolett-Färbung der Zellen. Nach Extraktion des Kristallviolett aus den fixierten Zellen wird die Absorption bei 550 nm spektralphotometrisch gemessen. Sie kann als quantitatives Maß für die vorhandenen adhärenten Zellen und damit der Zellproliferation während der Kultur herangezogen werden.

### In vitro-(Enzym-)Assay zur Bestimmung der Wirksamkeit der Inhibitoren der Hemmung von TGF-beta vermittelten Wirkungen

Der Kinaseassay wird als 384-well Flashplate assay durchgeführt. 31.2 nM GST-ALK5, 439 nM GST-SMAD2 und 3 mM ATP (mit 0.3µCi ³³P-ATP/well) werden in einem Gesamtvolumen von 35µl (20 mM HEPES, 10 mM MgCl, 5 mM MnCl, 1 mM DTT, 0.1 % BSA, pH 7.4) ohne oder mit Prüfsubstanz (5-10 Konzentrationen) für 45 Min bei 30°C inkubiert. Die Reaktion wird mit 25µl 200 mM EDTA-Lösung gestoppt, nach 30 Min bei Raumtemperatur abgesaugt und die Wells mit 3mal 100 µl 0.9%-ige NaCl-Lösung gewaschen. Radioaktivität wird im Topcount gemessen. Die IC₅₀-Werte werden mit RS1 berechnet.

**Tabelle 1: Inhibierung von TGF-beta**

| Verbindung Nr. | IC₅₀ [mol/l] |
|---|---|
| "A30" | 6,3 E-08 |
| "A53" | 2,4 E-07 |
| "A16" | 2,9 E-07 |
| "A41" | 1.8 E-07 |

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ |
| APCI-MS (atmospheric pressure chemical ionization - mass spectrometry) (M+H)⁺. | |

### Retentionszeit Rₜ [min] : Bestimmung erfolgt mit HPLC

Säule: Chromolith SpeedROD, 50 x 4.6 mm² (Best.Nr. 1.51450.0001) von Merck
Gradient: 5.0 min, t = 0 min, A:B = 95:5, t = 4.4 min: A:B = 25:75, t = 4.5 min bis t = 5.0 min: A:B = 0:100
Fluß: 3.00 ml/min
Laufmittel A: Wasser + 0, 1 % TFA (Trifluoressigsäure),
Laufmittel B: Acetonitril + 0,08% TFA
Wellenlänge: 220 nm

### LC-MS Bedingungen

Hewlett Packard System der HP 1100 Serie mit den folgenden Merkmalen: Ionenquelle: Elektrospray (positive mode); Scan: 100-1000 m/z; Fragmentier-Spannung: 60 V; Gas-Temperatur: 300°C, DAD: 220 nm.

Flussrate: 2.4 ml/Min. Der verwendete Splitter reduzierte nach dem DAD die Flussrate für das MS auf 0,75ml/Min.
Säule: Chromolith SpeedROD RP-18e 50-4.6
Lösungsmittel: LiChrosolv-Qualität der Fa. Merck KGaA
Lösungsmittel A: H2O (0.01 % TFA)
Lösungsmittel B: ACN (0.008% TFA)

### Gradient:

20% B → 100% B: 0 min bis 2.8 min
100% B: 2.8 min bis 3.3 min
100%B → 20%B: 3.3 min bis 4 min

Die in den nachfolgenden Beispielen angegebenen Retentionszeiten R_{f} [min] und M+H⁺-Daten MW sind die Meßergebnisse der LC-MS-Messungen.

### Beispiel 1

Allgemeines Reaktionsschema zur Herstellung von Verbindungen der Formel I, worin R² und R³ jeweils H bedeutet:

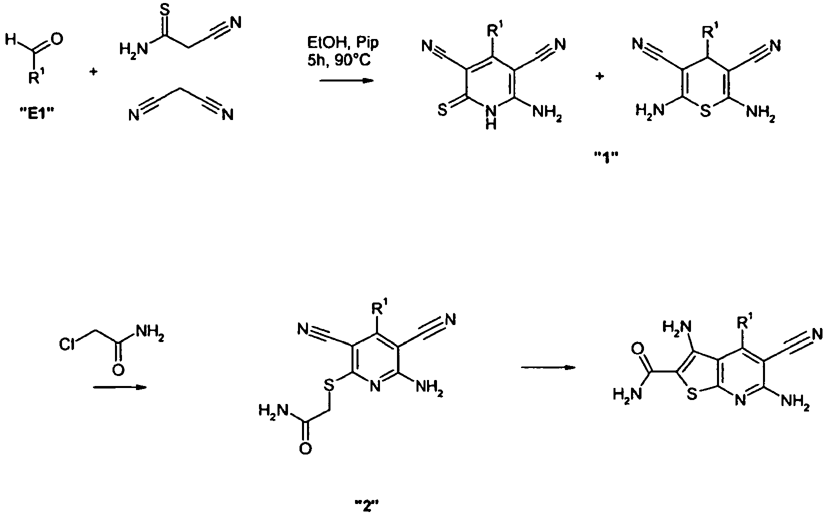

### Herstellung von 3,6-Diamino-4-benzofuran-2-yl-5-cyano-thieno[2,3-b]pyridin-2-carbonsäureamid ("A1"):

1.1 Eine Lösung von 6,0 g Benzofuran-2-carboxyaldehyd ("E1") in 200 ml Ethanol wird zunächst mit 2,685 g Malonsäuredinitril und 4,196 g Cyanothioacetamid versetzt. Hierauf wird 203,2 µl Piperidin zugegeben, die erhaltene rotbraune Suspension drei Stunden bei 90°C gekocht und anschließend 16 Stunden bei Raumtemperatur gerührt. Das ausgefallene Material (gelbe und orangefarbene Kristalle) werden abgetrennt, mit Ethanol und Dichlormethan gewaschen und getrocknet. Man erhält 8,1530 g von einem Gemisch aus 6-Amino-4-benzofuran-2-yl-2-thioxo-1,2-dihydro-pyridin-3,5-dicarbonitril und 2,6-Diamino-4-benzofuran-2-yl-2H-thiopyran-3,5-dicarbonitril ("1"), das ohne Aufreinigung weiter umgesetzt werden kann.
1.2 Eine Lösung von 1,0 g des entsprechend 1.1 hergestellten Gemisches ("1 ") in 15 ml DMF wird mit einem Äquivalent wässriger Kaliliauge und 320 mg 2-Chloracetamid versetzt. Nach einer Stunde wird nochmals eine Äquivalent Kalilauge zugegeben und 16 Stunden bei Raumtemperatur gerührt. Der Niederschlag wird abgetrennt, mit Wasser und Dichlormethan gewaschen und getrocknet. Man erhält 192 mg weißes Pulver von 2-(6-Amino-4-benzofuran-2-yl-3,5-dicyano-pyridin-2-ylsulfanyl)-acetamid ("2").
1.3 433 mg "2" wird in 10 ml DMF gelöst, mit 483 µl 10%iger wässriger Kalilauge KOH versetzt und vier Stunden bei Raumtemperatur gerührt, wobei sich die Lösung nach dunkelrot verfärbt. Anschließend wird Scherbeneis zugegeben, wobei sich ein orangefarbener Niederschlag bildet. Der Niederschlag wird abgetrennt, mit Wasser gewaschen und getrocknet. Man erhält 325,8 mg orangerotes Pulver von 3,6-Diamino-4-benzofuran-2-yl-5-cyano-thieno[2,3-b]pyridin-2-carbonsäureamid ("A1").
   1 H-NMR (500 MHz, DMSO-d6) ö(ppm):7.85 (1 H, d), 7.77 (1H, d), 7.55 (1 H, s), 7.5 (1 H, t), 7.47 (2H, br, NH2), 7.42 (1 H, t), 7.09 (2H, br, NH2), 6.19 (2H, br, NH2). 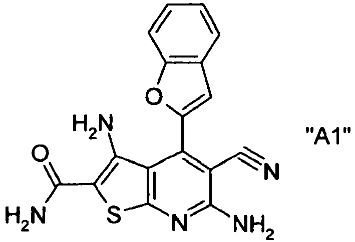 Analog erhält man bei Austausch von "E1" mit

### Benzofuran-2-carbaldehyd "A6", 5-Methyl-2-vinyl-benzofuran "A9",

| Nr. | Struktur und Name | MW |
|---|---|---|
| "A6" | 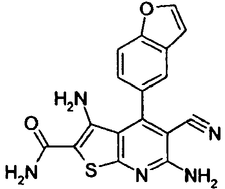 | 349,4 |
| | 3,6-Diamino-4-benzofuran-5-yl-5-cyano-thieno[2,3-b]pyridin-2-carbonsäureamid | |
| | HPLC-MS: [M+H] 350 1 H-NMR (500 MHz, DMSO-d6) δ(ppm): 8.15 1H, d), 7.91 (1 H, s), 7.80 (1 H, m), 7.41 (1 H, m), 7.28 (2H, br, NH2), 7.09 (1H, m), 6.94 (2H, br, NH2), 5.57 (2H, br, NH2) | |
| "A9" | 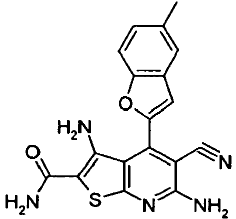 | 363,4 |
| | 3,6-Diamino-5-cyano-4-(5-methyl-benzofuran-2-yl)-thieno[2,3-b]pyridin-2-carbonsäureamid | |
| | HPLC-MS: [M+H] 364 1H-NMR (500 MHz, DMSO-d6) δ(ppm): 7.63 1H, s), 7.61 (1 H, m), 7.46 (1 H, s), 7.44 (2H, br, NH2), 7.29 (1H, m), 7.08 (2H, br, NH2), 6.17 (2H, br, NH2), 2.45 (3H, s, CH3) | |

### Beispiel 2

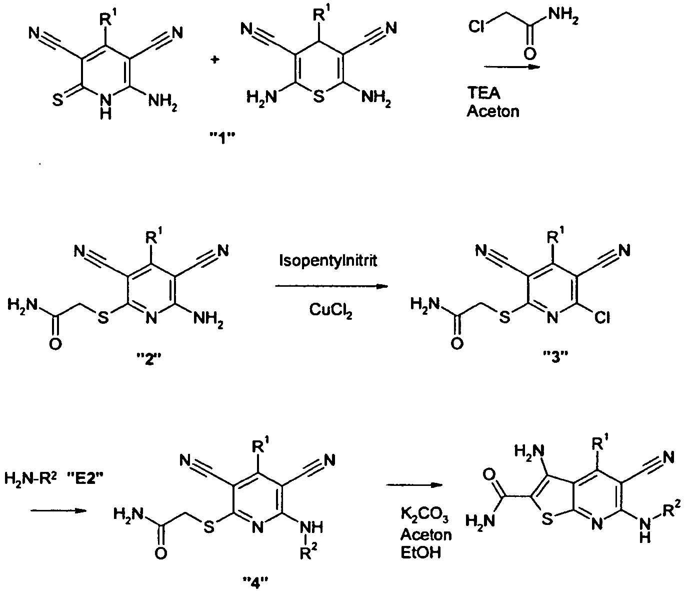

### Herstellung von 3-Amino-4-benzofuran-2-yl-5-cyano-6-[3-(4-methylpiperazin-1-yl)-propylamino]-thieno[2,3-b]pyridin-2-carbonsäureamid ("A30"):

2.1 Unter Inertatmosphäre werden einer Lösung von 3,97 g Kupfer(II)-chlorid und 4,96 ml Isopentylnitrit 470 ml trockenem Acetonitril 8,14 g ("2") zugegeben. Die erhaltene Suspension wird auf 65°C erhitzt und 4 Stunden gerührt. Die entstehende rotbraune Lösung wird auf Raumtemperatur abgekühlt, in 400 ml Salzsäure (20 Gew-%) überführt und dreimal mit je 100 ml Essigester extrahiert. Die vereinten organischen Phasen werden eingeengt und in Eiswasser überführt. Der erhaltene Niederschlag wird abgetrennt, mit Acetonitril und Wasser gewaschen und getrocknet.
   Man erhält 3,1326 g sandfarbenes Pulver von 2-(4-Benzofuran-2-yl-6-chloro-3,5-dicyano-pyridin-2-ylsulfanyl)-acetamid ("3").
   Gehalt HPLC: 95,8 %
   HPLC-MS: [M+H] 369
2.2 200 mg 2-(4-Benzofuran-2-yl-6-chloro-3,5-dicyano-pyridin-2-ylsulfanyl)-acetamid ("3") und 66 µl 1-(3-Aminopropyl)-4-methylpiperazin ("E2") in 2 ml Ethanol werden 16 Stunden bei Raumtemperatur gerührt. Anschließend werden 66 µl 1-(3-Aminopropyl)-4-methylpiperazin und 2 ml Ethanol zugegeben und für weitere 12 Stunden bei Raumtemeperatur weitergerührt. Es entsteht ein Niederschlag, der abgetrennt, mit Ethanol gewaschen und getrocknet wird.
   Man erhält 236,1 mg hellgelbes Pulver von 2-{4-Benzofuran-2-yl-3,5-dicyano-6-[3-(4-methyl-piperazi n-1-yl)-propylamino]-pyridin-2-ylsulfanyl}-acetamid ("4")..
   Gehalt HPLC: 93,2 %
   HPLC-MS: [M+H] 490
2.3 Eine Suspension von 236 mg 2-{4-Benzofuran-2-yl-3,5-dicyano-6-[3-(4-methyl-piperazin-1-yl)-propylamino]-pyridin-2-ylsulfanyl}-acetamids ("4") in einem Gemisch aus 7 ml wasserfreiem Aceton und 7 ml Ethanol absolut werden mit 124 mg Kaliumcarbonat versetzt, auf 65 °C erhitzt und mehrere Stunden gerührt. Anschließend wird über 16 Stunden abgekühlt, das Kaliumcarbonat abgetrennt und das Lösungsmittel entfernt. Der erhaltene Rückstand wird nacheinander mit Ethanol, Dichlormethan und Petroleumbenzin gewaschen.
   Man erhält 112,6 mg rotes Pulver von 3-Amino-4-benzofuran-2-yl-5-cyano-6-[3-(4-methyl-piperazin-1-yl)-propylamino]-thieno[2,3-b]pyridin-2-carbonsäureamid ("A30"). 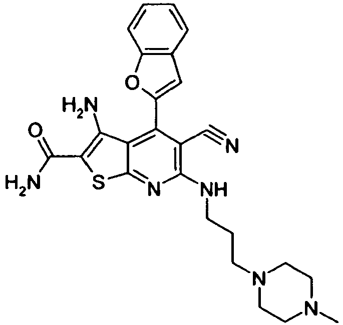 Gehalt HPLC: 95,5 %
   HPLC-MS: [M+H] 490
   1 H-NMR (500 MHz, DMSO-d6) δ(ppm): 7.83 (1 H, d), 7.73 (1H, d), 7.67 (1 H, br, NH), 7.51 (1 H, d), 7.48 (1H, m), 7.40 (1H, m), 7.05 (2H, br, NH2), 6.15 (2H, br, NH2), 3.61 (4H, m), 3.35 (4H, m), 2.95 (3H, s, CH3), 2.05 (4H, m), 1.09 (2H, m)

Analog erhält man bei Austausch von "E2" mit
Butylamin "A31 ",
4-Amino-butylamin "A32",
Morpholin "A33",
4-Methylpiperazin "A34",
3-Aminopropyl-piperidin "A35",
3-Morpholin-4-yl-propylamin "A36",
4-(4-Methyl-3-oxo-piperazin-1-yl)-piperidin "A37",
2-(2-hydroxy-ethoxy)-ethylamin "A38",
2-(4-methyl-piperazin-1-yl)-ethylamin "A39",
3-hydroxy-pyrrolidin "A40",
2,3-dihydroxy-propylamin "A41",
(3-hydroxy-cyclobutylmethyl)-amin "A42",
4-Ethansulfonyl-piperazin "A43",
(Tetrahydro-pyran-4-ylmethyl)-amin "A44",
(Tetrahydro-furan-2-ylmethoxy)-ethylamin "A45",
4-(4-methyl-piperazin-1-yl)-butylamin "A46",
6-(4-methyl-piperazin-1-yl)-hexylamin "A47",
2-Methylamino-ethylamin "A48",
4-Pyrrolidin-1-yl-piperidin "A49",
3-Imidazol-1-yl-propylamin "A50".

| Nr. | Struktur und Name | MW |
|---|---|---|
| "A31" | 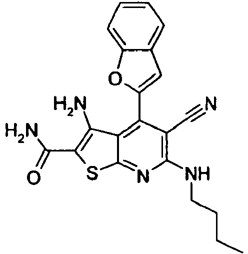 | 405,5 |
| | 3-Amino-4-benzofuran-2-yl-6-butylam ino-5-cyano-thieno[2,3-b]pyridin-2-carbonsäureamid | |
| | HPLC-MS: [M+H] 406 | |
| "A32" | 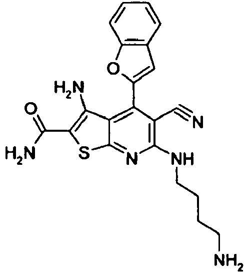 | 420,5 |
| | 3-Amino-6-(4-amino-butylamino)-4-benzofuran-2-yl-5-cyano-thieno[2,3-b]pyridin-2-carbonsäureamid | |
| | HPLC-MS: [M+H] 421 | |
| "A33" | 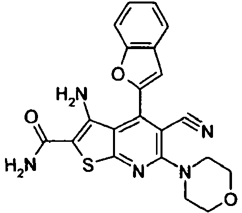 | 419,5 |
| | 3-Amino-4-benzofuran-2-yl-5-cyano-6-morpholin-4-yl-thieno[2,3-b]pyridin-2-carbonsäureamid | |
| | HPLC-MS: [M+H] 420 1H-NMR (500 MHz, DMSO-d6) δ(ppm): 7.84 (1H, d), 7.75 (1H, d), 7.57 (1 H, s), 7.49 (1 H, m), 7.40 (1 H, m), 7.21 (2H, br, NH2), 6.23 (2H, br, NH2), 3.76 (4H, m), 3.68 (4H, m) | |
| "A34" | 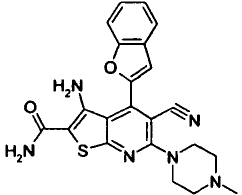 | 432,5 |
| | 3-Amino-4-benzofuran-2-yl-5-cyano-6-(4-methyl-piperazin-1-yl)-thieno[2, 3-b]pyridin-2-carbonsäureamid | |
| | HPLC-MS: [M+H] 433 | |
| "A35" | 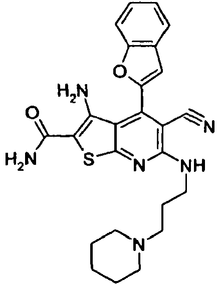 | 474,6 |
| | 3-Amino-4-benzofuran-2-yl-5-cyano-6-(3-piperidin-1-yl-propylamino)-thieno[2,3-b]pyridin-2-carbonsäureamid | |
| | HPLC-MS: [M+H] 475 | |
| "A36" | 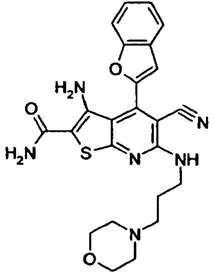 | 476,6 |
| | 3-Amino-4-benzofuran-2-yl-5-cyano-6-(3-morpholin-4-yl-propylamino)-thieno[2,3-b]pyridin-2-carbonsäureamid | |
| | HPLC-MS: [M+H] 477 | |
| "A37" | 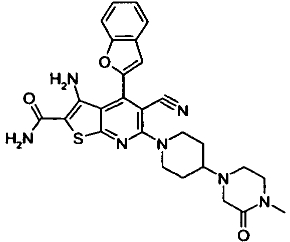 | 529,6 |
| | 3-Amino-4-benzofuran-2-yl-5-cyano-6-[4-(4-methyl-3-oxo-piperazin-1-yl)-piperidin-1-yl]-thieno[2,3-b]pyridin-2-carbonsäureamid | |
| | HPLC-MS: [M+H] 530 | |
| "A38" | 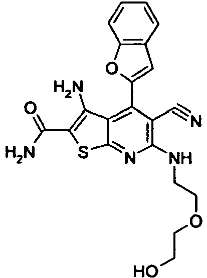 | 437,5 |
| | 3-Amino-4-benzofuran-2-yl-5-cyano-6-[2-(2-hydroxy-ethoxy)-ethylamino]-thieno[2,3-b]pyridin-2-carbonsäureamid | |
| | HPLC-MS: [M+H] 438 1H-NMR (500 MHz, DMSO-d6) δ(ppm): 7.83 (1H, d), 7.74 (1H, d), 7.54 (1H, d), 7.51 (1H, d), 7.48 (2H, m), 7.40 (1H, m), 7.08 (2H, br, NH2), 3.17 (2H, br, NH2), 4.6 (1H, br, OH), 3.63 (4H, m), 3.29 (4H, m) | |
| "A39" | 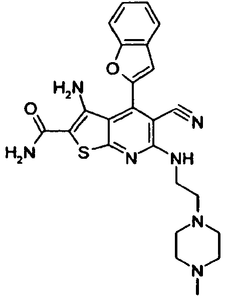 | 475,6 |
| | 3-Amino-4-benzofuran-2-yl-5-cyano-6-[2-(4-methyl-piperazin-1-yl)-ethylamino]-thieno[2,3-b]pyridin-2-carbonsäureamid | |
| | HPLC-MS: [M+H] 476 1H-NMR (500 MHz, DMSO-d6) δ(ppm): 7.84 (1 H, d), 7.75 (1H, d), 7.55 (1H, s), 7.50 (1H, m), 7.40 (1 H, m), 7.35 (1 H, br, NH), 7.08 (2H, br, NH2), 6.19 (2H, br, NH2), 3.55 (4H, m), 2.55 (4H, m), 2.46 (2H, br), 2.31 (2H, br), 2.14 (3H, s, CH3) | |
| "A40" | 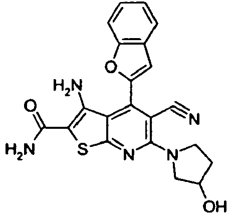 | 419,5 |
| | 3-Amino-4-benzofuran-2-yl-5-cyano-6-(3-hydroxy-pyrrolidin-1-yl)-thieno[2,3-b]pyridin-2-carbonsäureamid | |
| | HPLC-MS: [M+H] 420 | |
| "A41" | 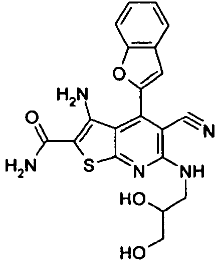 | 423,5 |
| | 3-Amino-4-benzofuran-2-yl-5-cyano-6-(2,3-dihydroxy-propylamino)-thieno[2,3-b]pyridin-2-carbonsäureamid | |
| | HPLC-MS: [M+H] 424 1H-NMR (500 MHz, DMSO-d6) δ(ppm): 7.84 (1H, d), 7.75 (1H, d), 7.55 (1H, br, NH), 7.48 1H, m), 7.41 (1H, m), 7.17 (1H, m, OH), 7.09 (2H, br, NH2), 6.18 (2H, br, NH2), 4.93 (1H, m, 3H), 4.66 (1H, m, OH), 3.76 (1H, m), 3.59 (1H, m), 3.41 (3H, m) | |
| "A42" | 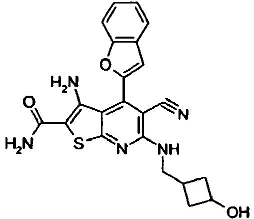 | 433,5 |
| | 3-Amino-4-benzofuran-2-yl-5-cyano-6-[(3-hydroxy-cyclobutylmethyl)-amino]-thieno[2,3-b]pyridin-2-carbonsäureamid | |
| | HPLC-MS: [M+H] 434 1 H-NMR (500 MHz, DMSO-d6) δ(ppm): 7.83 (1H, d), 7.75 (1 H, d), 7.66 (1H, br, NH), 7.54 (1H, s), 7.48 (1H, t), 7.40 (1H, t), 7.09 (2H, br, NH2), 6.15 (2H, br, NH2), 5.0 (1H, br, OH), 3.88 (1H, m), 3.45 (2H, m), 2.24 (2H, m), 2.05 (1H, m), 1.58 (2H, m) | |
| "A43" | 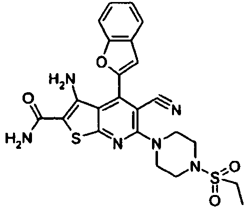 | 510,6 |
| | 3-Amino-4-benzofuran-2-yl-5-cyano-6-(4-ethansulfonyl-piperazin-1-yl)-thieno[2,3-b]pyridin-2-carbonsäureamid | |
| | HPLC-MS: [M+H] 511 1H-NMR (500 MHz, DMSO-d6) ö(ppm): 7.85 (1 H, d), 7.76 (1H, d), 7.59 (1 H, s), 7.50 (1H, t), 7.41 (1 H, t), 7.26 (2H, br, NH2), 6.27 (2H, br, NH2), 3.75 (4H, m), 3.38 (4H, m), 3.13 (2H, q, CH2), 1.23 (3H, t, CH3) | |
| "A44" | 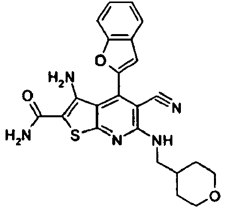 | 447,5 |
| | 3-Amino-4-benzofuran-2-yl-5-cyano-6-[(tetrahydro-pyran-4-ylmethyl)-amino]-thieno[2,3-b]pyridin-2-carbonsäureamid | |
| | HPLC-MS: [M+H] 448 1H-NMR (500 MHz, DMSO-d6) δ(ppm): 7.83 (1H, d), 7.75 (1H, d), 7.66 (1H, br, NH), 7.54 (1H, s), 7.48 (1H, t), 7.40 (1 H, t), 7.09 (2H, br, NH2), 6.16 (2H, br, NH2), 3.87 (2H, m), 3.40 (2H, m), 3.29 (2H, m), 1.98 (1H, m), 1.64 (2H, m), 1.27 (2H, m) | |
| "A45" | 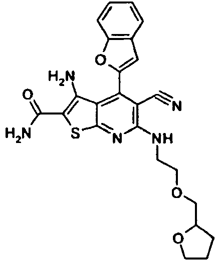 | 477,5 |
| | 3-Amino-4-benzofuran-2-yl-5-cyano-6-[2-(tetrahydro-furan-2-ylmethoxy)-ethylamino]-thieno[2,3-b]pyridin-2-carbonsäureamid | |
| | HPLC-MS: [M+H] 478 | |
| "A46" | 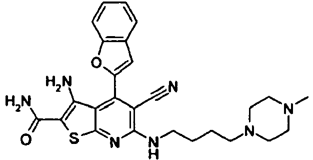 | 503,6 |
| | 3-Amino-4-benzofuran-2-yl-5-cyano-6-[4-(4-methyl-piperaz in-1-yl)-butylamino]-thieno[2, 3-b]pyridin-2-carbonsäureamid | |
| | HPLC-MS: [M+H] 504 | |
| "A47" | 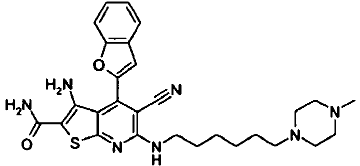 | 531,7 |
| | 3-Amino-4-benzofuran-2-yl-5-cyano-6-[6-(4-methyl-piperazin-1-yl)-hexylamino]-thieno[2,3-b]pyridin-2-carbonsäureamid | |
| | HPLC-MS: [M+H] 532 | |
| "A48" | 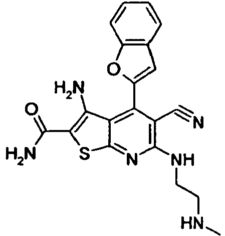 | 406,5 |
| | 3-Amino-4-benzofuran-2-yl-5-cyano-6-(2-methylamino-ethylamino)-thieno[2,3-b]pyridin-2-carbonsäureamid | |
| | HPLC-MS: [M+H] 407 | |
| "A49" | 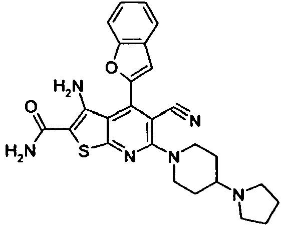 | 486,6 |
| | 3-Amino-4-benzofuran-2-yl-5-cyano-6-(4-pyrrolidin-1-yl-piperidin-1-yl)-thieno[2,3-b]pyridin-2-carbonsäureamid | |
| | HPLC-MS: [M+H] 487 | |
| "A50" | 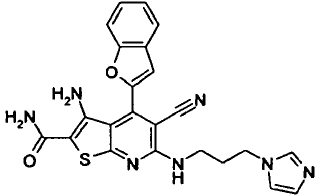 | 457,5 |
| | 3-Amino-4-benzofuran-2-yl-5-cyano-6-(3-imidazol-1-yl-propylamino)-thieno[2,3-b]pyridin-2-carbonsäureamid | |
| | HPLC-MS: [M+H] 459 1H-NMR (500 MHz, DMSO-d6) δ(ppm): 9.13 (1H, s), 7.85 (1H, d), 7.82 (1H, m), 7.76 (1H, d), 7.72 (1 H, m), 7.70 (1H, m), 7.54 (1H, m), 7.50 (1H, m), 7.42 (1H, m), 7.10 (2H, br, NH2), 6.18 (2H, br, NH2), 4.30 (2H, m), 3.51 (2H, m), 2.20 (2H, m) | |

### Beispiel 6

2-(6-Amino-4-benzofuran-2-yl-3,5-dicyano-pyridin-2-ylsulfanyl)-acetamid ("2"), das nach den Schritten 1.1 und 1.2 von Beispiel 1 erhältlich ist, wird analog den Schritten 2.1 bis 2.3 von Beispiel 2 umgesetzt. Bei Austausch von "E2" in Schritt 2.2 erhält man mit
3-Amino-propylamin "A71",
3-Ethansulfonylamino-propylamin "A72",
3-Hydroxy-propylamin "A73",
3-Methoxy-propylamin "A74°,
3-(2-Dimethylamino-ethoxy)-propylamin "A75",
3-(4-Dimethylamino-butoxy)-propylamin "A76",
2-Hydroxy-ethylamin "A77",
2-Hydroxymethyl-cyclopropylmethylamin "A78",
cis-2-Hydroxymethyl-cyclopropylmethylamin "A79"
Piperidin-4-yl-methylamin "A80",

| Nr. | Struktur und Name | MW |
|---|---|---|
| "A70" | 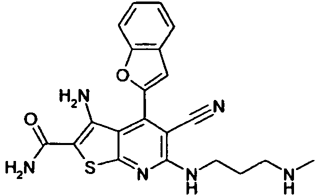 | 420,5 |
| | 3-Amino-4-benzofuran-2-yl-5-cyano-6-(3-methylamino-propylamino)-thieno[2,3-b]pyridin-2-carbonsäureamid | |
| | HPLC-MS: [M+H] 421 1H-NMR (500 MHz, DMSO-d6) ö(ppm): 8.61 (2H, br, NH2), 7.84 (1H, d), 7.75 (1H, d), 7.71 (1H, t, NH), 7.54 (1H, s), 7.49 (1H, t), 7.41 (1H, t), 7.10 (2H, br, NH2), 4.5 (1H, br, NH), 3.55 (2H, m), 2.96 (2H, m), 2.50 (3H, s, CH3), 1.96 (2H, m) | |
| "A71" | 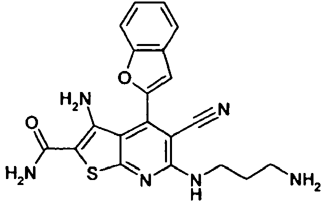 | 406,5 |
| | 3-Amino-6-(3-amino-propylamino)-4-benzofuran-2-yl-5-cyano-thieno[2,3-b]pyridin-2-carbonsäureamid | |
| | HPLC-MS: [M+H] 407 1H-NMR (500 MHz, DMSO-d6) ö(ppm): 7.85 (1H, d), 7.81 (2H, br, NH2), 7.76 (1H, d), 7.70 (1 H, t, NH), 7.55 (1 H, s), 7.50 (1H, m), 7.42 (1H, m), 7.12 (2H, br, NH2), 3.8 (2H, br, NH2), 3.55 (2H, m), 2.89 (2H, m), 1.93 (2H, m) | |
| "A72" | 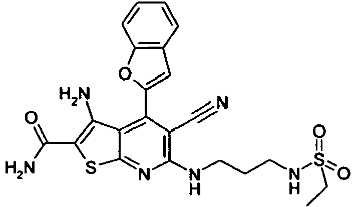 | 498,6 |
| | 3-Amino-4-benzofuran-2-yl-5-cyano-6-(3-ethansulfonylamino-propylamino)-thieno[2,3-b]pyridin-2-carbonsäureamid | |
| | HPLC-MS: [M+H] 499 1H-NMR (500 MHz, DMSO-d6) ö(ppm): 7.85 (1H, d), 7.77 (1H, d), 7.60 (1H, t, NH), 7.55 (1H, s), 7.50 (1H, t), 7.41 (1H, t), 7.10 (2H, br, NH2), 7.04 (1 H, t, NH), 6.17 (2H, br, NH2), 3.52 (2H, q), 3.02 (4H, m), 1.82 (2H, m), 1.21 (3H, t, CH3) | |
| "A73" | 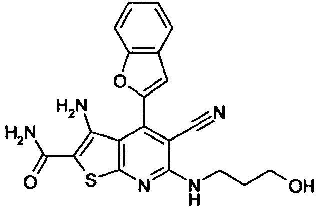 | 407,5 |
| | 3-Amino-4-benzofuran-2-yl-5-cyano-6-(3-hydroxy-propylamino)-thieno[2,3-b]pyridin-2-carbonsäureamid | |
| | HPLC-MS: [M+H] 408 | |
| "A74" | 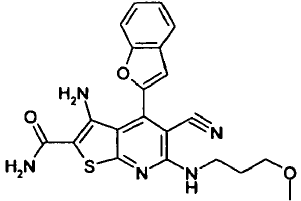 | 421,5 |
| | 3-Amino-4-benzofuran-2-yl-5-cyano-6-(3-methoxy-propylamino)-thieno[2,3-b]pyridin-2-carbonsäureamid | |
| | HPLC-MS: [M+H] 422 1H-NMR (500 MHz, DMSO-d6) δ(ppm): 7.85 (1H, d), 7.77 (1H, d), 7.60 (1 H, t, NH), 7.50 (1H, t), 7.41 (1H, t), 7.09 (2H, br, NH2), 6.17 (2H, br, NH2), 3.52 (2H, m), 3.43 (2H, m), 3.26 (3H, s, CH3), 1.86 (2H, m) | |
| "A75" | 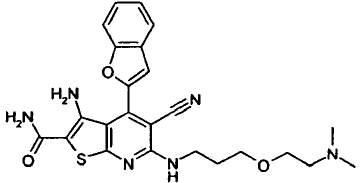 | 478,6 |
| | 3-Amino-4-benzofuran-2-yl-5-cyano-6-[3-(2-dimethylamino-ethoxy)-propylamino]-thieno[2,3-b]pyridin-2-carbonsäureamid | |
| | HPLC-MS: [M+H] 479 1H-NMR (500 MHz, DMSO-d6) δ(ppm): 7.84 (1H, d), 7.76 (1 H, d), 7.56 (2H, m + s), 7.50 (1H, t), 7.41 (1H, t), 7.10 (2H, br, NH2), 6.18 (2H, br, NH2), 3.71 (2H, m), 3.55 (4H, m), 3.30 (2H, m), 2.81 (6H, s, NCH3), 1.91 (2H, m) | |
| "A76" | 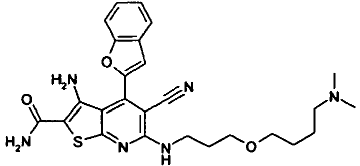 | 506,6 |
| | 3-Amino-4-benzofuran-2-yl-5-cyano-6-[3-(4-dimethylamino-butoxy)-propylamino]-thieno[2,3-b]pyridin-2-carbonsäureamid | |
| | HPLC-MS: [M+H] 507 1H-NMR (500 MHz, DMSO-d6) δ(ppm): 7.84 (1H, d), 7.75 (1H, d), 7.53 (2H, br + s), 7.48 (1 H, t), 7.40 (1 H, t), 7.07 (2H, br, NH2), 6.16 (2H, br, NH2), 3.53 (2H, m), 3.46 (2H, m), 3.37 (2H, m), 2.16 (2H, m), 2.07 (6H, s, NCH3), 1.85 (2H, m), 1.49 (2H, m), 1.41 (2H, m) | |
| "A77" | 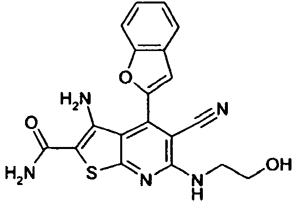 | 393,4 |
| | 3-Amino-4-benzofuran-2-yl-5-cyano-6-(2-hydroxy-ethylamino)-thieno[2,3-b]pyridin-2-carbonsäureamid | |
| | HPLC-MS: [M+H] 394 | |
| "A78" | 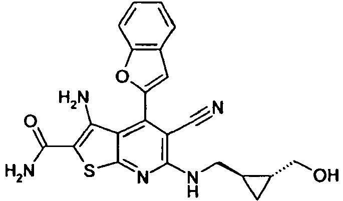 | 433,5 |
| | 3-Amino-4-benzofuran-2-yl-5-cyano-6-[trans-(2-hydroxymethyl-cyclopropylmethyl)-amino]-thieno[2,3-b]pyridin-2-carbonsäureamid | |
| | HPLC-MS: [M+H] 434 1H-NMR (500 MHz, DMSO-d6) δ(ppm): 7.85 (1 H, d), 7.76 (1H, d), 7.65 (1 H, t, NH), 7.56 (1 H, s), 7.50 (1 H, t), 7.41 (1H, t), 7.11 (2H, br, NH2), 6.17 (2H, br, NH2), 4.48 (1H, t, OH), 3.33 (3H, m), 3.18 (1H, m), 1.04 (1H, m), 0.96 (1H, m), 0.46 (1H, m), 0.35 (1H, m) | |
| "A79" | 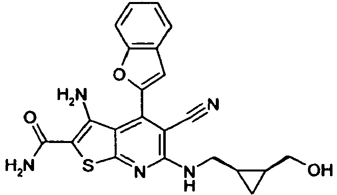 | 433,5 |
| | 3-Amino-4-benzofuran-2-yl-5-cyano-6-[cis-(2-hydroxymethyl-cyclopropylmethyl)-amino]-thieno[2,3-b]pyridin-2-carbonsäureamid | |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 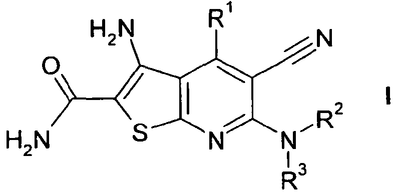 worin
R¹ Het
R², R³ jeweils unabhängig voneinander H, A, AlkNH₂, AlkNHA, AlkNAA', AlkNHSO₂A, AlkOH, AlkOA, AlkCyc, AlkCycAlkOH, AlkCycAlkOA, AlkCycAlkC0OA, AlkCycAlkC0OH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ oder AlkAr, wobei einer der Substituenten R2 und R3 ≠ H ist,
R² und R³ zusammen auch eine Alkylenkette mit 1 bis 6 C-Atomen sein kann, worin eine oder zwei nicht benachbarte CH₂-Gruppen durch N- und/oder O-Atome und/oder worin 1 bis 6 H-Atome durch A, OH, OA, (CH₂)ₙHet¹, SO₂A und/oder Hal ersetzt sein können,
Alk Alkylen mit 1 bis 6 C-Atomen, worin 1 bis 4 H-Atome durch F, Cl und/oder Br ersetzt sein können,
Cyc Cycloalkyl mit 3 bis 7 C-Atomen, worin 1 bis 4 H-Atome durch A, Hal, OH und/oder OA ersetzt sein können,
Het Benzofuranyl, das ein- oder zweifach durch A substituiert sein kann,
Het' einen einkernigen gesättigten, ungesättigten oder aromatischen Heterocyclus mit 1 bis 4 N-, O- und/oder S-Atomen, der ein-, zwei- oder dreifach durch A, OH, OA, Hal, SO₂A und/oder =O (Carbonylsauerstoff) substituiert sein kann,
Ar Phenyl, das unsubstituiert oder ein-, zwei- oder dreifach durch A, OH, OA, Hal, SO₂NH₂, SO₂NA und/oder SO₂NAA' substituiert ist,
A, A' jeweils unabhängig voneinander unverzweigtes oder verzweigtes Alkyl mit 1-10 C-Atomen, worin 1-5 H-Atome durch F, Cl und/oder Br ersetzt sein können,
Hal F, Cl, Br oder I,
m 1, 2, 3, 4,
n 0, 1, 2, 3, 4
bedeuten, .
sowie ihre pharmazeutisch verwendbaren Salze, Solvate, Tautomere und Stereoisomere, einschließlich deren Mischungen in allen Verhältnissen.

2. Verbindungen nach Anspruch 1,
worin
R² H, A, AlkNH₂, AlkNHA, AlkNAA', AlkNHSO₂A, AlkOH, AlkOA, AlkCyc, AlkCycAlkOH, AlkCycAlkOA, AlkCycAlkCOOA, AlkCycAlkCOOH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ oder AlkAr und
R³ H bedeutet und
sowie ihre pharmazeutisch verwendbaren Solvate, Salze und Stereoisomere, einschließlich deren Mischungen in allen Verhältnissen.

3. Verbindungen nach Anspruch 1 oder 2,
worin
R² und R³ zusammen auch eine Alkylenkette mit 1 bis 5 C-Atomen sein kann, worin eine nicht benachbarte CH₂-Gruppe durch ein N- oder O-Atom und/oder worin 1 oder 2 H-Atome durch A, OH, OA, (CH₂)ₙHet¹, und/oder SO₂A ersetzt sein können,
sowie ihre pharmazeutisch verwendbaren Solvate, Salze und Stereoisomere, einschließlich deren Mischungen in allen Verhältnissen.

4. Verbindungen nach einem oder mehreren der Ansprüche 1 bis 3,
worin
Het¹ einen einkernigen gesättigten oder ungesättigten Heterocyclus mit 1 bis 3 N-, O- und/oder S-Atomen, der ein-, zwei- oder dreifach durch A, Hal, SO₂A und/oder =O (Carbonylsauerstoff) substituiert sein kann,
sowie ihre pharmazeutisch verwendbaren Solvate, Salze und Stereoisomere, einschließlich deren Mischungen in allen Verhältnissen.

5. Verbindungen nach einem oder mehreren der Ansprüche 1 bis 4, worin
Ar Phenyl, das einfach durch SO₂NH₂, SO₂NA oder SO₂NAA' substituiert ist, bedeutet
sowie ihre pharmazeutisch verwendbaren Solvate, Salze und Stereoisomere, einschließlich deren Mischungen in allen Verhältnissen.

6. Verbindungen ausgewählt aus der Gruppe
3,6-Diamino-4-benzofuran-2-yl-5-cyano-thieno[2,3-b]pyridin-2-carbonsäureamid,
3,6-Diamino-4-benzofuran-5-yl-5-cyano-thieno[2,3-b]pyridin-2-carbonsäureamid,
3,6-Diamino-5-cyano-4-(5-methyl-benzofuran-2-yl)-thieno[2,3-b]pyridin-2-carbonsäureamid,
3-Amino-4-benzofuran-2-yl-5-cyano-6-[3-(4-methyl-piperazin-1-yl)-propylamino]-thieno[2,3-b]pyridin-2-carbonsäureamid,
3-Amino-6-(4-amino-butylamino)-4-benzofuran-2-yl-5-cyano-thieno[2,3-b]pyridin-2-carbonsäureamid,
33-Amino-4-benzofuran-2-yl-5-cyano-6-morpholin-4-yl-thieno[2,3-b]pyridin-2-carbonsäureamid,
3-Amino-4-benzofuran-2-yl-5-cyano-6-(4-methyl-piperazin-1-yl)-thieno[2,3-b]pyridin-2-carbonsäureamid,
3-Amino-4-benzofuran-2-yl-5-cyano-6-(3-piperidin-1-yl-propylamino)-thieno[2,3-b]pyridin-2-carbonsäureamid,
3-Amino-4-benzofuran-2-yl-5-cyano-6-(3-morpholin-4-yl-propylamino)-thieno[2,3-b]pyridin-2-carbonsäureamid,
3-Amino-4-benzofuran-2-yl-5-cyano-6-[4-(4-methyl-3-oxo-piperazin-1-yl)-piperidin-1-yl]-thieno[2,3-b]pyridin-2-carbonsäureamid,
3-Amino-4-benzofuran-2-yl-5-cyano-6-[2-(2-hydroxy-ethoxy)-ethylamino]-thieno[2,3-b]pyridin-2-carbonsäureamid,
3-Amino-4-benzofuran-2-yl-5-cyano-6-[2-(4-methyl-piperazin-1-yl)-ethylamino]-thieno[2,3-b]pyridin-2-carbonsäureamid,
3-Amino-4-benzofuran-2-yl-5-cyano-6-(3-hydroxy-pyrrolidin-1-yl)-thieno[2,3-b]pyridin-2-carbonsäureamid,
3-Amino-4-benzofuran-2-yl-5-cyano-6-(2,3-dihydroxy-propylamino)-thieno[2,3-b]pyridin-2-carbonsäureamid,
3-Amino-4-benzofuran-2-yl-5-cyano-6-[(3-hydroxy-cyclobutylmethyl)-amino]-thieno[2,3-b]pyridin-2-carbonsäureamid,
3-Amino-4-benzofuran-2-yl-5-cyano-6-(4-ethansulfonyl-piperazin-1-yl)-thieno[2,3-b]pyridin-2-carbonsäureamid,
3-Amino-4-benzofuran-2-yl-5-cyano-6-[(tetrahydro-pyran-4-ylmethyl)-amino]-thieno[2,3-b]pyridin-2-carbonsäureamid,
3-Amino-4-benzofuran-2-yl-5-cyano-6-[2-(tetrahydro-furan-2-ylmethoxy)-ethylamino]-thieno[2,3-b]pyridin-2-carbonsäureamid,
3-Amino-4-benzofuran-2-yl-5-cyano-6-[6-(4-methyl-piperazin-1-yl)-hexylamino]-thieno[2,3-b]pyridin-2-carbonsäureamid,
3-Amino-4-benzofuran-2-yl-5-cyano-6-[6-(4-methyl-piperazin-1-yl)-hexylamino]-thieno[2,3-b]pyridin-2-carbonsäureamid,
3-Amino-4-benzofuran-2-yl-5-cyano-6-(2-methylamino-ethylamino)-thieno[2,3-b]pyridin-2-carbonsäureamid,
3-Amino-4-benzofuran-2-yl-5-cyano-6-(4-pyrrolidin-1-yl-piperidin-1-yl)-thieno[2,3-b]pyridin-2-carbonsäureamid,
3-Amino-4-benzofuran-2-yl-5-cyano-6-(3-imidazol-1-yl-propylamino)-thieno[2,3-b]pyridin-2-carbonsäureamid,
3-Amino-4-benzofuran-2-yl-5-cyano-6-(3-methylamino-propylamino)-thieno[2,3-b]pyridin-2-carbonsäureamid,
3-Amino-6-(3-amino-propylamino)-4-benzofuran-2-yl-5-cyano-thieno[2,3-b]pyridin-2-carbonsäureamid,
3-Amino-4-benzofuran-2-yl-5-cyano-6-(3-ethansulfonylamino-propylamino)-thieno[2,3-b]pyridin-2-carbonsäureamid,
3-Amino-4-benzofuran-2-yl-5-cyano-6-(3-hydroxy-propylamino)-thieno[2,3-b]pyridin-2-carbonsäureamid,
3-Amino-4-benzofuran-2-yl-5-cyano-6-(3-methoxy-propylamino)-thieno[2,3-b]pyridin-2-carbonsäureamid,
3-Amino-4-benzofuran-2-yl-5-cyano-6-[3-(2-dimethylamino-ethoxy)-propylamino]-thieno[2,3-b]pyridin-2-carbonsäureamid,
3-Amino-4-benzofuran-2-yl-5-cyano-6-[3-(4-dimethylamino-butoxy)-propylamino]-thieno[2,3-b]pyridin-2-carbonsäureamid,
3-Amino-4-benzofuran-2-yl-5-cyano-6-(2-hydroxy-ethylamino)-thieno[2,3-b]pyridin-2-carbonsäureamid,
3-Amino-4-benzofuran-2-yl-5-cyano-6-[trans-(2-hydroxymethyl-cydopropylmethyl)-amino]-thieno[2,3-b]pyridin-2-carbonsäureamid,
3-Amino-4-benzofuran-2-yl-5-cyano-6-[cis-(2-hydroxymethyl-cyclopropylmethyl)-amino]-thieno[2,3-b]pyridin-2-carbonsäureamid,
sowie ihre pharmazeutisch verwendbaren Solvate, Salze und Stereoisomere, einschließlich deren Mischungen in allen Verhältnissen.

7. Verfahren zur Herstellung von Verbindungen der Formel I nach den Ansprüchen 1 bis 7 sowie ihrer pharmazeutisch verwendbaren Salze, Solvate, Tautomeren und Stereoisomeren, **dadurch gekennzeichnet, daß** man
a) zur Herstellung einer Verbindung der Formel I worin R², R³ = H sind,
eine Verbindung der Formel II 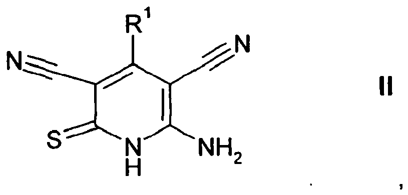 worin R¹ die in Anspruch 1 angegebene Bedeutung hat,
mit einer Verbindung der Formel III
H₂N-CO-CH₂-Z III
worin
Z Cl, Br, I oder eine freie oder reaktionsfähige funktionell abgewandelte OH-Gruppe bedeutet,
zu einer Verbindung der Formel IV 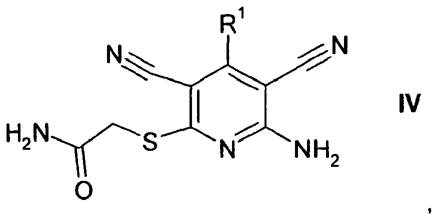 worin R¹ die in Anspruch 1 angegebene Bedeutung hat,
umsetzt,
und die erhaltene Verbindung der Formel IV anschließend zur Verbindung der Formel I zyklisiert,
oder
b) zur Herstellung der Verbindung der Formel I, worin mindestens einer der beiden Reste R², R³ ≠ H ist,
in einer Verbindung der Formel IV die freie Aminogruppe gegen Hal austauscht,
wobei eine Verbindung der Formel V 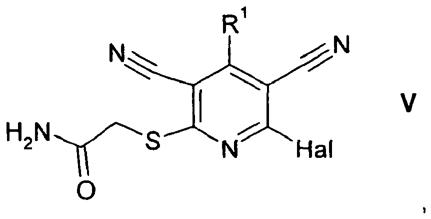 worin R¹ die in Anspruch 1 angegebene Bedeutung hat,
erhalten wird,
die Verbindung der Formel V mit einer Verbindung der Formel VI 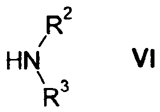 worin R² und R³ die in Anspruch 1 angegebenen Bedeutungen haben, jedoch mindestens einer der beiden Reste R², R³ ≠ H ist,
zu einer Verbindung der Formel VII 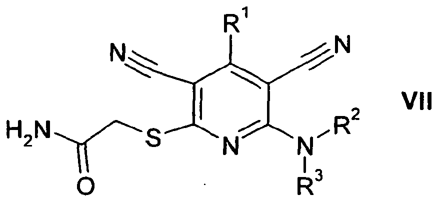 worin R¹, R², R³ die in Anspruch 1 angegebenen Bedeutungen haben, jedoch mindestens einer der beiden Reste R², R³ ≠ H ist,
umsetzt
und die erhaltene Verbindung der Formel VII anschließend zur Verbindung der Formel I zyklisiert,
und/oder
c) eine Base oder Säure der Formel I in eines ihrer Salze umwandelt.

8. Arzneimittel, enthaltend mindestens eine Verbindung nach einem oder mehreren der Ansprüche 1 bis 6 und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

9. Verwendung von Verbindungen gemäß einem oder mehreren der Ansprüche 1 bis 6 sowie ihrer pharmazeutisch verwendbaren Salze, Solvate, Tautomeren und Stereoisomeren, einschließlich deren Mischungen in allen Verhältnissen,
zur Herstellung eines Arzneimittels zur Behandlung und/oder Bekämpfung von Krebs, Tumorwachstum, Metastasenwachstum, Fibrose, Restenose, HIV Infektion, Alzheimer, Atherosklerose, und/oder zur Förderung der Wundheilung.

10. Verwendung nach Anspruch 9, wobei der Tumor ausgewählt ist aus der Gruppe der Tumoren des Plattenepithel, der Blasen, des Magens, der Nieren, von Kopf und Hals, des Ösophagus, des Gebärmutterhals, der Schilddrüse, des Darms, der Leber, des Gehirns, der Prostata, des Urogenitaltrakts, des lymphatischen Systems, des Magens, des Kehlkopfs, der Lunge, Lungenadenokarzinom, kleinzelliges Lungenkarzinom, Bauchspeicheldrüsenkrebs, Glioblastom, Kolonkarzinom, Brustkarzinom, Tumor des Blut- und Immunsystems, akute myelotische Leukämie, chronische myelotische Leukämie, akute lymphatische Leukämie, chronische lymphatische Leukämie.

11. Verbindungen gemäß einem oder mehreren der Ansprüche 1 bis 6 und/oder ihrer physiologisch unbedenklichen Salze und Solvate zur Behandlung von festen Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

12. Verbindungen gemäß einem oder mehreren der Ansprüche 1 bis 6 und/oder ihrer physiologisch unbedenklichen Salze und Solvate zur Behandlung von festen Tumoren wobei eine therapeutisch wirksame Menge einer Verbindung gemäß einem oder mehreren der Ansprüche 1 bis 7 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

13. Arzneimittel enthaltend mindestens eine Verbindung gemäß einem oder mehreren der Ansprüche 1 bis 7 und/oder ihre pharmazeutisch verwendbaren Solvate und Stereoisomere, einschließlich deren Mischungen in allen Verhältnissen, und mindestens einen weiteren Arzneimittelwirkstoff.

14. Set (Kit), bestehend aus getrennten Packungen von
(a) einer wirksamen Menge an einer Verbindung der gemäß einem oder mehreren Anspüchen 1 bis 7 und/oder ihrer pharmazeutisch verwendbaren Solvate und Stereoisomere, einschließlich deren Mischungen in allen Verhältnissen,
und
(b) einer wirksamen Menge eines weiteren Arzneimittelwirkstoffs.

## Claims

1. Compounds of the formula I 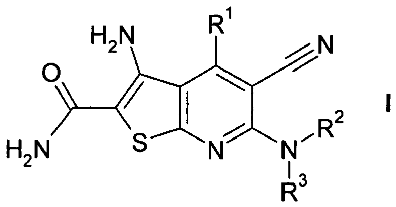 in which
R¹ denotes Het
R², R³ each, independently of one another, denote H, A, AlkNH₂, AlkNHA, AlkNAA', AlkNHSO₂A, AlkOH, AlkOA, AlkCyc, AlkCycAlkOH, AlkCycAlkOA, AlkCycAlkCOOA, AlkCycAlkCOOH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ or AlkAr, where one of the substituents R2 and R3 is ≠ H,
R² and R³ together may also be an alkylene chain having 1 to 6 C atoms, in which one or two non-adjacent CH₂ groups may be replaced by N and/or O atoms and/or in which 1 to 6 H atoms may be replaced by A, OH, OA, (CH₂)ₙHet¹, SO₂A and/or Hal,
Alk denotes alkylene having 1 to 6 C atoms, in which 1 to 4 H atoms may be replaced by F, CI and/or Br,
Cyc denotes cycloalkyl having 3 to 7 C atoms, in which 1 to 4 H atoms may be replaced by A, Hal, OH and/or OA,
Het denotes benzofuranyl, which may be mono-, di- or trisubstituted by A,
Het¹ denotes a monocyclic saturated, unsaturated or aromatic heterocycle having 1 to 4 N, O and/or S atoms, which may be mono-, di- or trisubstituted by A, OH, OA, Hal, SO₂A and/or =O (carbonyl oxygen),
Ar denotes phenyl which is unsubstituted or mono-, di- or trisubstituted by A, OH, OA, Hal, SO₂NH₂, SO₂NA and/or SO₂NAA',
A, A' each, independently of one another, denote unbranched or branched alkyl having 1-10 C atoms, in which 1-5 H atoms may be replaced by F, Cl and/or Br,
Hal denotes F, Cl, Br or I,
m denotes 1, 2, 3, 4,
n denotes 0, 1, 2, 3, 4,
and pharmaceutically usable salts, solvates, tautomers and stereoisomers thereof, including mixtures thereof in all ratios.

2. Compounds according to Claim 1
in which
R² denotes H, A, AlkNH₂, AlkNHA, AlkNAA', AlkNHSO₂A, AlkOH, AlkOA, AlkCyc, AlkCycAlkOH, AlkCycAlkOA, AlkCycAlkCOOA, AlkCycAlkCOOH, AlkHet¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHet¹ or AlkAr and
R³ denotes H,
and pharmaceutically usable solvates, salts and stereoisomers thereof, including mixtures thereof in all ratios.

3. Compounds according to Claim 1 or 2
in which
R² and R³ together may also be an alkylene chain having 1 to 5 C atoms, in which one non-adjacent CH₂ group may be replaced by an N or O atom and/or in which 1 or 2 H atoms may be replaced by A, OH, OA, (CH₂)ₙHet¹, and/or SO₂A,
and pharmaceutically usable solvates, salts and stereoisomers thereof, including mixtures thereof in all ratios.

4. Compounds according to one or more of Claims 1 to 3
in which
Het¹ denotes a monocyclic saturated or unsaturated heterocycle having 1 to 3 N, O and/or S atoms, which may be mono-, di- or trisubstituted by A, Hal, SO₂A and/or =O (carbonyl oxygen),
and pharmaceutically usable solvates, salts and stereoisomers thereof, including mixtures thereof in all ratios.

5. Compounds according to one or more of Claims 1 to 4
in which
Ar denotes phenyl which is monosubstituted by SO₂NH₂, SO₂NA or SO₂NAA'
and pharmaceutically usable solvates, salts and stereoisomers thereof, including mixtures thereof in all ratios.

6. Compounds selected from the group
3,6-diamino-4-benzofuran-2-yl-5-cyanothieno[2,3-b]pyridine-2-carboxamide,
3,6-diamino-4-benzofuran-5-yl-5-cyanothieno[2,3-b]pyridine-2-carboxamide,
3,6-diamino-5-cyano-4-(5-methylbenzofuran-2-yl)thieno[2,3-b]-pyridine-2-carboxamide,
3-amino-4-benzofuran-2-yl-5-cyano-6-[3-(4-methylpiperazin-1-yl)propylamino]thieno[2,3-b]pyridine-2-carboxamide,
3-amino-6-(4-aminobutylamino)-4-benzofuran-2-yl-5-cyano-thieno[2,3-b]pyridine-2-carboxamide,
3-amino-4-benzofuran-2-yl-5-cyano-6-morpholin-4-ylthieno-[2,3-b]pyridine-2-carboxamide,
3-amino-4-benzofuran-2-yl-5-cyano-6-(4-methylpiperazin-1-yl)-thieno[2,3-b]pyridine-2-carboxamide,
3-amino-4-benzofuran-2-yl-5-cyano-6-(3-piperidin-1-ylpropyl-amino)thieno[2,3-b]pyridine-2-carboxamide,
3-amino-4-benzofuran-2-yl-5-cyano-6-(3-morpholin-4-ylpropyl-amino)thieno[2,3-b]pyridine-2-carboxamide,
3-amino-4-benzofuran-2-yl-5-cyano-6-[4-(4-methyl-3-oxo-piperazin-1-yl)piperidin-1-yl]thieno[2,3-b]pyridine-2-carboxamide,
3-amino-4-benzofuran-2-yl-5-cyano-6-(2-(2-hydroxyethoxy)-ethylamino]thieno[2,3-b]pyridine-2-carboxamide,
3-amino-4-benzofuran-2-yl-5-cyano-6-[2-(4-methylpiperazin-1-yl)ethylamino]thieno[2,3-b]pyridine-2-carboxamide,
3-amino-4-benzofuran-2-yl-5-cyano-6-(3-hydroxypyrrolidin-1-yl)-thieno[2,3-b]pyridine-2-carboxamide,
3-amino-4-benzofuran-2-yl-5-cyano-6-(2,3-dihydroxypropyl-amino)thieno[2,3-b]pyridine-2-carboxamide,
3-amino-4-benzofuran-2-yl-5-cyano-6-[(3-hydroxycyclobutyl-methyl)amino]thieno[2,3-b]pyridine-2-carboxamide,
3-amino-4-benzofuran-2-yl-5-cyano-6-(4-ethanesulfonyl-piperazin-1-yl)thieno[2,3-b]pyridine-2-carboxamide,
3-amino-4-benzofuran-2-yl-5-cyano-6-[(tetrahydropyran-4-yl-methyl)amino]thieno[2,3-b]pyridine-2-carboxamide,
3-amino-4-benzofuran-2-yl-5-cyano-6-[2-(tetrahydrofuran-2-yl-methoxy)ethylamino]thieno[2,3-b]pyridine-2-carboxamide,
3-amino-4-benzofuran-2-yl-5-cyano-6-[6-(4-methylpiperazin-1-yl)hexylamino]thieno[2,3-b]pyridine-2-carboxamide,
3-amino-4-benzofuran-2-yl-5-cyano-6-[6-(4-methylpiperazin-1-yl)hexylamino]thieno[2,3-b]pyridine-2-carboxamide,
3-amino-4-benzofuran-2-yl-5-cyano-6-(2-methylaminoethyl-amino)thieno[2,3-b]pyridine-2-carboxamide,
3-amino-4-benzofuran-2-yl-5-cyano-6-(4-pyrrolidin-1-ylpiperidin-1-yl)thieno[2,3-b]pyridine-2-carboxamide,
3-amino-4-benzofuran-2-yl-5-cyano-6-(3-imidazol-1-ylpropyl-amino)thieno[2,3-b]pyridine-2-carboxamide,
3-amino-4-benzofuran-2-yl-5-cyano-6-(3-methylaminopropyl-amino)thieno[2,3-b]pyridine-2-carboxamide,
3-amino-6-(3-aminopropylamino)-4-benzofuran-2-yl-5-cyano-thieno[2,3-b]pyridine-2-carboxamide,
3-amino-4-benzofuran-2-yl-5-cyano-6-(3-ethanesulfonylamino-propylamino)thieno[2,3-b]pyridine-2-carboxamide,
3-amino-4-benzofuran-2-yl-5-cyano-6-(3-hydroxypropylamino)-thieno[2,3-b]pyridine-2-carboxamide,
3-amino-4-benzofuran-2-yl-5-cyano-6-(3-methoxypropylamino)-thieno[2,3-b]pyridine-2-carboxamide,
3-amino-4-benzofuran-2-yl-5-cyano-6-[3-(2-dimethylamino-ethoxy)propylamino]thieno[2,3-b]pyridine-2-carboxamide,
3-amino-4-benzofuran-2-yl-5-cyano-6-[3-(4-dimethylamino-butoxy)propylamino]thieno[2,3-b]pyridine-2-carboxamide,
3-amino-4-benzofuran-2-yl-5-cyano-6-(2-hydroxyethylamino)-thieno[2,3-b]pyridine-2-carboxamide,
3-amino-4-benzofuran-2-yl-5-cyano-6-[trans-(2-hydroxymethyl-cyclopropylmethyl)amino]thieno[2,3-b]pyridine-2-carboxamide,
3-amino-4-benzofuran-2-yl-5-cyano-6-[cis-(2-hydroxymethyl-cyclopropylmethyl)amino]thieno[2,3-b]pyridine-2-carboxamide,
and pharmaceutically usable solvates, salts and stereoisomers thereof, including mixtures thereof in all ratios.

7. Process for the preparation of compounds of the formula I according to Claims 1 to 6 and pharmaceutically usable salts, solvates, tautomers and stereoisomers thereof, **characterised in that**
a) for the preparation of a compound of the formula I in which R², R³ = H,
a compound of the formula II 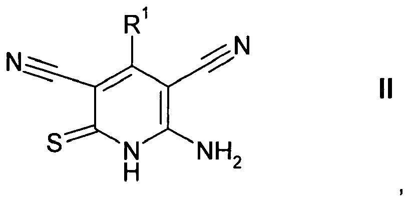 in which R¹ has the meaning indicated in Claim 1,
is reacted with a compound of the formula III
H₂N-CO-CH₂-Z III
in which
Z denotes Cl, Br, I or a free or reactive functionally modified OH group,
to give a compound of the formula IV 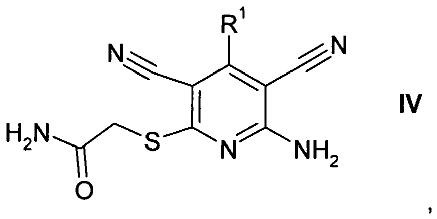 in which R¹ has the meaning indicated in Claim 1,
and the resultant compound of the formula IV is subsequently cyclised to give the compound of the formula I,
or
b) for the preparation of the compound of the formula I in which at least one of the two radicals R², R³ is ≠ H,
the free amino group in a compound of the formula IV is replaced by Hal,
giving a compound of the formula V 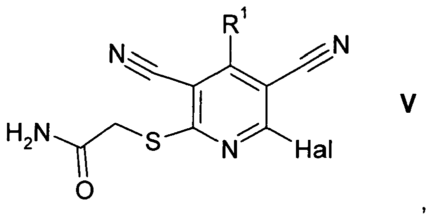 in which R¹ has the meaning indicated in Claim 1,
the compound of the formula V is reacted with a compound of the formula VI 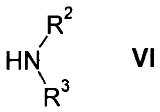 in which R² and R³ have the meanings indicated in Claim 1, but at least one of the two radicals R², R³ is ≠ H,
to give a compound of the formula VII 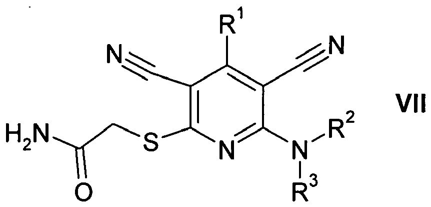 in which R¹, R², R³ have the meanings indicated in Claim 1, but at least one of the two radicals R², R³ is ≠ H,
and the resultant compound of the formula VII is subsequently cyclised to give the compound of the formula I, and/or
c) a base or acid of the formula I is converted into one of its salts.

8. Medicament comprising at least one compound according to one or more of Claims 1 to 6 and/or pharmaceutically usable solvates, salts and stereoisomers thereof, including mixtures thereof in all ratios, and optionally excipients and/or adjuvants.

9. Use of compounds according to one or more of Claims 1 to 6 and pharmaceutically usable salts, solvates, tautomers and stereoisomers thereof, including mixtures thereof in all ratios,
for the preparation of a medicament for the treatment and/or combating of cancer, tumour growth, metastatic growth, fibrosis, restenosis, HIV infection, Alzheimer's, atherosclerosis, and/or for promoting wound healing.

10. Use according to Claim 9, where the tumour is selected from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, lung adenocarcinoma, small-cell lung carcinoma, pancreatic cancer, glioblastoma, colon carcinoma, breast carcinoma, tumour of the blood and immune system, acute myeloid leukaemia, chronic myeloid leukaemia, acute lymphatic leukaemia, chronic lymphatic leukaemia.

11. Compounds according to one or more of Claims 1 to 6 and/or physiologically acceptable salts and solvates thereof for the treatment of solid tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

12. Compounds according to one or more of Claims 1 to 6 and/or physiologically acceptable salts and solvates thereof for the treatment of solid tumours, where a therapeutically effective amount of a compound according to one or more of Claims 1 to 6 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

13. Medicament comprising at least one compound according to one or more of Claims 1 to 6 and/or pharmaceutically usable solvates and stereoisomers thereof, including mixtures thereof in all ratios, and at least one further medicament active compound.

14. Set (kit) consisting of separate packs of
(a) an effective amount of a compound according to one or more of Claims 1 to 6 and/or pharmaceutically usable solvates and stereoisomers thereof, including mixtures thereof in all ratios,
and
(b) an effective amount of a further medicament active compound.

## Revendications

1. Composés de formule I 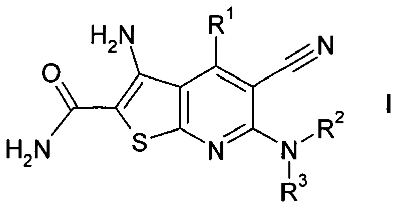 dans laquelle
R¹ désigne Hét
R², R³ désignent chacun, indépendamment l'un de l'autre, H, A, AlkNH₂, AlkNHA, AlkNAA', AlkNHSO₂A, AlkOH, AlkOA, AlkCyc, AlkCycAlkOH, AlkCycAlkOA, AlkCycAlkCOOA, AlkCycAlkCOOH, AlkHét¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHét¹ ou AlkAr, où l'un des substituants R2 et R3 est ≠ H,
R² et R³ peuvent aussi être ensemble une chaîne alkylène ayant de 1 à 6 atomes de C, dans laquelle un ou deux groupements CH₂ non adjacents peuvent être remplacés par des atomes de N et/ou O et/ou dans laquelle de 1 à 6 atomes de H peuvent être remplacés par A, OH, OA, (CH₂)ₙHét¹, SO₂A et/ou Hal,
Alk désigne alkylène ayant de 1 à 6 atomes de C, où de 1 à 4 atomes de H peuvent être remplacés par F, Cl et/ou Br,
Cyc désigne cycloalkyle ayant de 3 à 7 atomes de C, où de 1 à 4 atomes de H peuvent être remplacés par A, Hal, OH et/ou OA,
Hét désigne benzofuranyle, pouvant être mono-, di- ou trisubstitué par A,
Hét¹ désigne un hétérocycle monocyclique saturé, insaturé ou aromatique ayant 1 à 4 atomes de N, O et/ou S, pouvant être mono-, di- ou trisubstitué par A, OH, OA, Hal, SO₂A et/ou =O (oxygène de carbonyle),
Ar désigne phényle qui est non substitué ou mono-, di- ou trisubstitué par A, OH, OA, Hal, SO₂NH₂, SO₂NA et/ou SO₂NAA',
A, A' désignent chacun, indépendamment l'un de l'autre, alkyle non ramifié ou ramifié ayant 1-10 atomes de C, où 1-5 atomes de H peuvent être remplacés par F, CI et/ou Br,
Hal désigne F, CI, Br ou I,
m désigne 1, 2, 3, 4,
n désigne 0, 1, 2, 3, 4,
et les sels, solvats, tautomères et stéréoisomères pharmaceutiquement utilisables de ceux-ci, y compris des mélanges de ceux-ci en toutes proportions.

2. Composés selon la revendication 1,
dans lesquels
R² désigne H, A, AlkNH₂, AlkNHA, AlkNAA', AlkNHSO₂A, AlkOH, AlkOA, AlkCyc, AlkCycAklOH, AlkCycAlkOA, AlkCycAlkCOOA, AlkCycAlkCOOH, AlkHét¹, AlkOAlkOH, AlkOAlkOA, AlkOAlkNH₂, AlkOAlkNHA, AlkOAlkNAA', AlkCHOH(CH₂)ₙOH, AlkO(CH₂)ₘHét¹ ou AlkAr et
R³ désigne H,
et les solvats, sels et stéréoisomères pharmaceutiquement utilisables de ceux-ci, y compris des mélanges de ceux-ci en toutes proportions.

3. Composés selon la revendication 1 ou 2,
dans lesquels
R² et R³ peuvent aussi être ensemble une chaîne alkylène ayant 1 à 5 atomes de C, dans laquelle un groupement CH₂ non adjacent peut être remplacé par un atome de N ou O et/ou dans laquelle 1 ou 2 atomes de H peuvent être remplacés par A, OH, OA, (CH₂)ₙHét¹, et/ou SO₂A,
et les solvats, sels et stéréoisomères pharmaceutiquement utilisables de ceux-ci, y compris des mélanges de ceux-ci en toutes proportions.

4. Composés selon l'une ou plusieurs parmi les revendications 1 à 3,
dans lesquels
Hét¹ désigne un hétérocycle monocyclique saturé ou insaturé ayant 1 à 3 atomes de N, O et/ou S, pouvant être mono-, di- ou trisubstitué par A, Hal, SO₂A et/ou =O (oxygène de carbonyle),
et les solvats, sels et stéréoisomères pharmaceutiquement utilisables de ceux-ci, y compris des mélanges de ceux-ci en toutes proportions.

5. Composés selon l'une ou plusieurs parmi les revendications 1 à 4, dans lesquels
Ar désigne phényle qui est monosubstitué par SO₂NH₂, SO₂NA ou SO₂NAA'
et les solvats, sels et stéréoisomères pharmaceutiquement utilisables de ceux-ci, y compris des mélanges de ceux-ci en toutes proportions.

6. Composés choisis dans le groupe constitué par
le 3,6-diamino-4-benzofuran-2-yl-5-cyanothiéno[2,3-b]pyridine-2-carboxamide,
le 3,6-diamino-4-benzofuran-5-yl-5-cyanothiéno[2,3-b]pyridine-2-carboxamide,
le 3,6-diamino-5-cyano-4-(5-méthylbenzofuran-2-yl)thiéno-[2,3-b]pyridine-2-carboxamide,
le 3-amino-4-benzofuran-2-yl-5-cyano-6-[3-(4-méthylpipérazin-1-yl)propylamino]thiéno[2,3-b]pyridine-2-carboxamide,
le 3-amino-6-(4-aminobutylamino)-4-benzofuran-2-yl-5-cyano-thiéno[2,3-b]pyridine-2-carboxamide,
le 3-amino-4-benzofuran-2-yl-5-cyano-6-morpholin-4-ylthiéno-[2,3-b]pyridine-2-carboxamide,
le 3-amino-4-benzofuran-2-yl-5-cyano-6-(4-méthylpipérazin-1-yl)thiéno[2,3-b]pyridine-2-carboxamide,
le 3-amino-4-benzofuran-2-yl-5-cyano-6-(3-pipéridin-1-ylpropyl-amino)thiéno[2,3-b]pyridine-2-carboxamide,
le 3-amino-4-benzofuran-2-yl-5-cyano-6-(3-morpholin-4-yl-propylamino)thiéno[2,3-b]pyridine-2-carboxamide,
le 3-amino-4-benzofuran-2-yl-5-cyano-6-[4-(4-méthyl-3-oxo-pipérazin-1-yl)pipéridin-1-yl]thiéno[2,3-b]pyridine-2-carboxamide,
le 3-amino-4-benzofuran-2-yl-5-cyano-6-[2-(2-hydroxyéthoxy)-éthylamino]thiéno[2,3-b]pyridine-2-carboxamide,
le 3-amino-4-benzofuran-2-yl-5-cyano-6-[2-(4-méthylpipérazin-1-yl)éthylamino]thiéno[2,3-b]pyridine-2-carboxamide,
le 3-amino-4-benzofuran-2-yl-5-cyano-6-(3-hydroxypyrrolidin-1-yl)thiéno[2,3-b]pyridine-2-carboxamide,
le 3-amino-4-benzofuran-2-yl-5-cyano-6-(2,3-dihydroxypropyl-amino)thiéno[2,3-b]pyridine-2-carboxamide,
le 3-amino-4-benzofuran-2-yl-5-cyano-6-[(3-hydroxycyclobutyl-méthyl)amino]thiéno[2,3-b]pyridine-2-carboxamide,
le 3-amino-4-benzofuran-2-yl-5-cyano-6-(4-éthanesulfonyl-pipérazin-1-yl)thiéno[2,3-b]pyridine-2-carboxamide,
le 3-amino-4-benzofuran-2-yl-5-cyano-6-[(tétrahydropyran-4-yl-méthyl)amino]thiéno[2,3-b]pyridine-2-carboxamide,
le 3-amino-4-benzofuran-2-yl-5-cyano-6-[2-(tétrahydrofuran-2-ylméthoxy)éthylamino]thiéno[2,3-b]pyridine-2-carboxamide,
le 3-amino-4-benzofuran-2-yl-5-cyano-6-[6-(4-méthylpipérazin-1-yl)hexylamino]thiéno[2,3-b]pyridine-2-carboxamide,
le 3-amino-4-benzofuran-2-yl-5-cyano-6-[6-(4-méthylpipérazin-1-yl)hexylamino]thiéno[2,3-b]pyridine-2-carboxamide,
le 3-amino-4-benzofuran-2-yl-5-cyano-6-(2-méthylaminoéthyl-amino)thiéno[2,3-b]pyridine-2-carboxamide,
le 3-amino-4-benzofuran-2-yl-5-cyano-6-(4-pyrrolidin-1-yl-pipéridin-1-yl)thiéno[2,3-b]pyridine-2-carboxamide,
le 3-amino-4-benzofuran-2-yl-5-cyano-6-(3-imidazol-1-ylpropyl-amino)thiéno[2,3-b]pyridine-2-carboxamide,
le 3-amino-4-benzofuran-2-yl-5-cyano-6-(3-méthylaminopropyl-amino)thiéno[2,3-b]pyridine-2-carboxamide,
le 3-amino-6-(3-aminopropylamino)-4-benzofuran-2-yl-5-cyano-thiéno[2,3-b]pyridine-2-carboxamide,
le 3-amino-4-benzofuran-2-yl-5-cyano-6-(3-éthanesulfonyl-aminopropylamino)thiéno[2,3-b]pyridine-2-carboxamide,
le 3-amino-4-benzofuran-2-yl-5-cyano-6-(3-hydroxypropyl-amino)thiéno[2,3-b]pyridine-2-carboxamide,
le 3-amino-4-benzofuran-2-yl-5-cyano-6-(3-méthoxypropyl-amino)thiéno[2,3-b]pyridine-2-carboxamide,
le 3-amino-4-benzofuran-2-yl-5-cyano-6-[3-(2-diméthylamino-éthoxy)propylamino]thiéno[2,3-b]pyridine-2-carboxamide,
le 3-amino-4-benzofuran-2-yl-5-cyano-6-[3-(4-diméthylamino-butoxy)propylamino]thiéno[2,3-b]pyridine-2-carboxamide,
le 3-amino-4-benzofuran-2-yl-5-cyano-6-(2-hydroxyéthylamino)-thiéno[2,3-b]pyridine-2-carboxamide,
le 3-amino-4-benzofuran-2-yl-5-cyano-6-[trans-(2-hydroxy-méthylcyclopropylméthyl)amino]thiéno[2,3-b]pyridine-2-carbox-amide,
le 3-amino-4-benzofuran-2-yl-5-cyano-6-[cis-(2-hydroxyméthyl-cyclopropylméthyl)amino]thiéno[2,3-b]pyridine-2-carboxamide,
et les solvats, sels et stéréoisomères pharmaceutiquement utilisables de ceux-ci, y compris des mélanges de ceux-ci en toutes proportions.

7. Procédé de préparation de composés de formule I selon les revendications 1 à 6 et de sels, solvats, tautomères et stéréoisomères pharmaceutiquement utilisables de ceux-ci, **caractérisé en ce que**
a) pour la préparation d'un composé de formule I dans laquelle R² R³ = H,
un composé de formule II 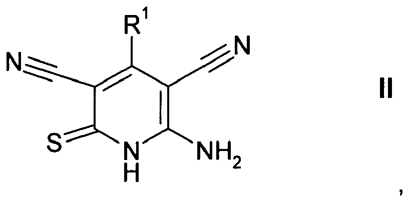 dans laquelle R¹ revêt la signification indiquée dans la revendication 1,
est réagi avec un composé de formule III
H₂N-CO-CH₂-Z **III**
dans laquelle
Z désigne CI, Br, I ou un groupement OH libre ou modifié de manière fonctionnelle et réactive,
pour donner un composé de formule IV 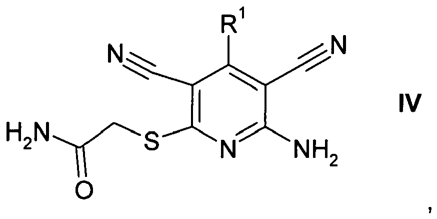 dans laquelle R¹ revêt la signification indiquée dans la revendication 1,
et le composé résultant de formule IV est ensuite cyclisé pour donner le composé de formule I,
ou
b) pour la préparation du composé de formule I dans laquelle au moins l'un parmi les radicaux R², R³ est # H,
le groupement amino libre dans un composé de formule IV est remplacé par Hal,
donnant un composé de formule V 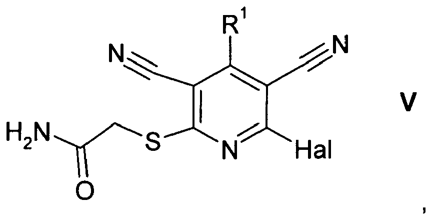 dans laquelle R¹ revêt la signification indiquée dans la revendication 1,
le composé de formule V est réagi avec un composé de formule VI 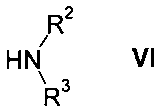 dans laquelle R² et R³ revêtent les significations indiquées selon la revendication 1, mais au moins l'un parmi les deux radicaux R², R³ est # H,
pour donner un composé de formule VII 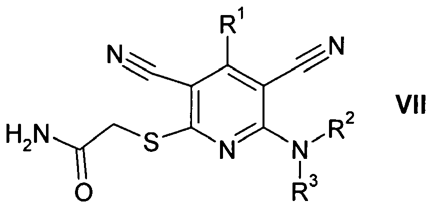 dans laquelle R¹, R², R³ revêtent les significations indiquées selon la revendication 1, mais au moins l'un parmi les deux radicaux R², R³ est # H,
et le composé résultant de formule VII est ensuite cyclisé pour donner le composé de formule I,
et/ou
c) une base ou un acide de formule I est converti(e) en l'un de ses sels.

8. Médicament comprenant au moins un composé selon l'une ou plusieurs parmi les revendications 1 à 6 et/ou des solvats, sels et stéréoisomères pharmaceutiquement utilisables de celui-ci, y compris des mélanges de ceux-ci en toutes proportions, et éventuellement des excipients et/ou des adjuvants.

9. Utilisation de composés selon l'une ou plusieurs parmi les revendications 1 à 6 et de sels, solvats, tautomères et stéréoisomères pharmaceutiquement utilisables de ceux-ci, y compris des mélanges de ceux-ci en toutes proportions,
pour la préparation d'un médicament destiné au traitement et/ou à la lutte contre le cancer, la croissance tumorale, la croissance métastatique, la fibrose, la resténose, l'infection par le VIH, la maladie d'Alzheimer, l'athérosclérose, et/ou destiné à favoriser la guérison de plaies.

10. Utilisation selon la revendication 9, dans laquelle la tumeur est choisie dans le groupe constitué par les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, l'adénocarcinome du poumon, les carcinomes du poumon à petites cellules, le cancer du pancréas, les glioblastomes, le carcinome du côlon, le carcinome du sein, une tumeur du sang et du système immunitaire, la leucémie myéloïde aiguë, la leucémie myéloïde chronique, la leucémie lymphatique aiguë, la leucémie lymphatique chronique.

11. Composés selon l'une ou plusieurs parmi les revendications 1 à 6 et/ou des sels et solvats physiologiquement acceptables de ceux-ci pour le traitement de tumeurs solides, où une quantité thérapeutiquement efficace d'un composé de formule I est administrée en association avec un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

12. Composés selon l'une ou plusieurs parmi les revendications 1 à 6 et/ou des sels et solvats physiologiquement acceptables de ceux-ci pour le traitement de tumeurs solides, où une quantité thérapeutiquement efficace d'un composé selon l'une ou plusieurs parmi les revendications 1 à 6 est administrée en association avec une radiothérapie et un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

13. Médicament comprenant au moins un composé selon l'une ou plusieurs parmi les revendications 1 à 6 et/ou des solvats et stéréoisomères pharmaceutiquement utilisables de ceux-ci, y compris des mélanges de ceux-ci en toutes proportions, et au moins un autre composé actif médicamenteux.

14. Ensemble (kit) constitué de conditionnements séparés
(a) d'une quantité efficace d'un composé selon l'une ou plusieurs parmi les revendications 1 à 6 et/ou de solvats et stéréoisomères pharmaceutiquement utilisables de ceux-ci, y compris des mélanges de ceux-ci en toutes proportions,
et
(b) d'une quantité efficace d'un autre composé actif médicamenteux.
